# EUROPEAN PATENT SPECIFICATION

(11) **EP 4 251 210 B1**
(45) Date of publication and mention of the grant of the patent: **19.03.2025**
(21) Application number: 21773652.9
(22) Date of filing: 24.08.2021
(51) Int. Cl.: A61K 47/69, A61K 47/65, A61K 47/68, A61P 35/00, C07K 16/30, C07K 16/32

(54) **MULTI-SPECIFIC HUMAN ALBUMIN NANOPARTICLES DECORATED WITH ANTIBODY FRAGMENTS AND LOADED WITH CYTOTOXICS**
MULTISPEZIFISCHE HUMANALBUMIN-NANOPARTIKEL, DIE MIT ANTIKÖRPERFRAGMENTEN DEKORIERT UND MIT ZYTOTOXISCHEN MITTELN BELADEN SIND
NANOPARTICULES MULTI-SPÉCIFIQUES D'ALBUMINE HUMAINE DÉCORÉES DE FRAGMENTS D'ANTICORPS ET CHARGÉES DE MÉDICAMENTS CYTOTOXIQUES

(30) Priority: 30.11.2020 IT 202000028952
(43) Date of publication of application: 04.10.2023
(73) Proprietor: Anbition S.r.l., 80123 Napoli (IT)
(72) Inventor: RUVO, Menotti, 81020 San Nicola La Strada (Caserta) (IT); DE FALCO, Sandro, 80136 Napoli (IT); ROSIELLO, Davide, 80128 Napoli (IT)
(74) Representative: Biggi, Cristina
(86) International application number: PCT/IB2021/057740
(87) International publication number: WO 2022/112865

(56) References cited:
- WO-A1-2022/112866
- KOUCHAKZADEH HASAN ET AL: "Optimization of an anti-HER2 monoclonal antibody targeted delivery system using PEGylated human serum albumin nanoparticles", INTERNATIONAL JOURNAL OF PHARMACEUTICS, vol. 447, no. 1-2, 27 February 2013 (2013-02-27), NL, pages 62 - 69, XP055828976, ISSN: 0378-5173, DOI: 10.1016/j.ijpharm.2013.02.043
- SELIS FABIO ET AL: "Generation and testing of engineered multimeric Fabs of trastuzumab", INTERNATIONAL JOURNAL OF BIOLOGICAL MACROMOLECULES, ELSEVIER BV, NL, vol. 164, 14 September 2020 (2020-09-14), pages 4516 - 4531, XP086334676, ISSN: 0141-8130, [retrieved on 20200914], DOI: 10.1016/J.IJBIOMAC.2020.09.050

## Description

### FIELD OF THE INVENTION

The present invention relates to serum albumin nanoparticles, preferably human, optionally loaded with a cytotoxic drug, decorated on the surface with at least one biological molecule capable of recognising over-expressed or selectively expressed target receptors on the surface of cells, preferably cancer cells.

### BACKGROUND OF THE INVENTION

Human albumin (HSA) is the most efficient and versatile natural carrier protein for drugs introduced into circulation and for small endogenous molecules. The bond of the molecules to HSA improves their pharmacokinetic profile, reducing the rapid excretion thereof through the renal system and toxicity. HSA is the most abundant plasma protein (35-50 g/L of human serum) and has a molecular weight of 66.5 kDa. Like most plasma proteins, it is synthesised in the liver where it is produced at a rate of about 0.7 mg/h per gram of liver (i.e., 10-15 g per day). HSA has a mean plasma half-life of 19 days. Its long half-life largely depends on its ability to bind to the FcRn receptor with high affinity [1]. HSA acts as a solubiliser for long-chain fatty acids and is therefore essential for lipid metabolism, binds bilirubin, the degradation product of heme, binds a large number of therapeutic drugs such as penicillins, sulphonamides, indolic compounds and benzodiazepines to name only a few [2]. It is able to complex metals such as copper (II) and nickel (II) in a specific manner and calcium (II) and zinc (II) in a relatively non-specific manner, acting as a transport vehicle in the blood also for these metal ions.

HSA is an acidic protein, very soluble and extremely stable; it is stable in the pH range comprised between 4 and 9, is soluble in 40% ethanol and can be heated at 60 °C even for 10 h without structural loss. These properties, together with its ability to be absorbed preferentially in tumour and inflamed tissues, its ready and wide availability, its biodegradability and its intrinsic lack of toxicity and immunogenicity make it the ideal molecule for the delivery of therapeutic drugs and other molecules associated therewith (payloads) in a covalent or non-covalent manner.

HSA is able to accumulate preferentially in tumour and inflamed tissues thanks to the presence therein of damaged capillaries and an absent or defective lymphatic drainage system. This property, known as "passive tumour targeting", exploits the increased ability of molecules with a molecular weight greater than about 40 kDa to permeate and be retained in tumour tissues thanks to the defective blood vessels which make the vascular walls permeable while those of the healthy tissue let only small molecules to pass through the endothelial barrier. The pore size of the tumour micro-vessels ranges from 100 to 1200 nm in diameter [3,4] while HSA has an effective diameter of 7.2 nm, which allows extravasation only in tumour tissue and not in normal tissue. This phenomenon leads to a greater uptake of macromolecules such as HSA in tumour tissue and favours the use thereof as a carrier for low molecular weight anticancer drugs which would normally be excreted much more quickly and would exert cytotoxic activity also in healthy tissues.

Albumin accumulation has been observed in many animal models of solid tumours including sarcoma, ovarian carcinoma and Novikof hepatoma [5]. It is also interesting to note that in models of syngeneic breast tumours, in excrescences of murine breast intraepithelial neoplasia and in tumours deriving from epithelial-mesenchymal transition, the permeability to albumin is ~ 4 times greater than that of liposomes or other similar synthetic nanoparticles of 100 nm in size [6], a phenomenon probably also favoured by the greater plasma half-life of HSA.

Thanks to all these properties, the conjugation to albumin of small molecules and therapeutic peptides or hormones such as insulin or small proteins such as cytokines is becoming a widely used and effective approach to improve their pharmacokinetic profile and quasi-specific transport towards diseased tissues.

In addition to passive accumulation in tumour tissues, HSA is also preferably internalised by many cancer cells, further favouring its use for the intracellular release of therapeutics associated therewith. In fact, it has been shown that cancer cells, through a mechanism of macropinocytosis, absorb and metabolise extracellular proteins such as HSA to meet their increased metabolic needs. It has also been observed that cancer cells expressing the oncogene Ras, an internal plasma membrane protein whose hyper-expression and hyper-activation is associated with basically all phenotypes of malignant cancer, use extracellular proteins more as a source of amino acids to support cell growth [7,8]. For example, pancreatic ductal adenocarcinoma cells are able to grow indefinitely in media free of essential amino acids but containing physiological concentrations of albumin [9] which is capable of being preferentially internalised by cancer cells with respect to normal cells. Hypoalbuminemia has also been observed as a common feature in patients with advanced solid tumours [10].

Some cancer cells, as well as through non-specific macropinocytic mechanisms, are able to internalise HSA also through receptor-mediated mechanisms. It has been shown that the preferential internalisation of HSA in cancer cells is mainly due to the binding with Caveolin, Cav-1, a protein abundantly present in the caveolae, a microdomain of cell membranes characterised by a peculiar lipid composition which allows specific molecules to cross the membranes, such as antibodies, complement factors and blood clotting factors, which are not able to cross them in any other manner, for example by filtration or diffusion.

Cav-1 is over-expressed in a wide variety of cancer types including pancreatic cancer, prostate cancer, and breast cancer, and the over-expression of Cav-1 is associated with the progression of cancer [11, 12].

Several approaches based on the use of HSA have been described for targeted cancer therapies. For example, it has been proposed to covalently bind therapeutics to HSA or load them in a non-covalent manner, by exploiting the affinity of specific protein binding sites for small molecules or for albumin-binding domains (ABD). Inserted in larger macromolecules such as nanobodies (NB, 15 kDa) or other proteins such as human TRAIL (30 kDa), ABD confer binding properties to the HSA, forming very stable complexes [13, 14]. The main method for covalent coupling on HSA instead exploits the presence of the amino acid cysteine 34 in reduced form, therefore with a thiol capable of selectively reacting with electrophilic groups.

A further commonly used strategy involves the conjugation of payloads on the side chains of lysine. This strategy exploits the formation of amide or imine bonds (or simple C-N bonds following any reductive amination reactions) and has the advantage, with respect to cysteine 34, of allowing the coupling of a greater number of payloads. However, the absence of selectivity of the reaction can compromise binding to the FcRn receptor, reducing the plasma half-life of the payload-HSA conjugate [15]. It should also be considered that it is difficult to control the number of changes and the specificity of the site through this reaction, thus obtaining molecules which are very heterogeneous from a structural point of view, difficult to characterise and to frame from a regulatory point of view.

Another widely used method for coupling small molecules to HSA exploits drug encapsulation in recombinant or naturally extracted protein-based nanoparticles. Human serum albumin nanoparticles (NP-HSAs) are known for this purpose. The advantage in using NP-HSAs lies in their ability to bind and/or trap a very large number of payloads which are released over time as a function of conditions. The payloads are thus retained in the bloodstream much longer, are protected from degradation, and can be more selectively unloaded to the therapeutic site of interest (e.g., in the tumour), reducing their interactions with healthy tissues.

Methods for synthesising albumin nanoparticles can generally be classified as desolvation, emulsification, thermal gelation, drying, and self-assembly techniques [2, 16]. The size of the NP-HSAs is a crucial parameter for their biological function, as particles which are too large in diameter would not be able to permeate the vessels of tumour tissue or interact with the caveolae. However, the specificity of HSA for tumour tissues is rather reduced when compared to that of molecules such as antibodies which recognise surface receptors with high selectivity and effectiveness. Thus, to confer further selectivity towards cancer cells, the albumin nanoparticles can be decorated with a variety of specific molecules (targeting agents or TAs) which recognise particular target receptors which are over-expressed or selectively expressed on the surface of cancer cells. NPs of mannosylated HSA were used for this purpose, to selectively target drug-resistant colon cancer cells and tumour-associated macrophages expressing high levels of mannose and SPARC receptors. In a similar approach, bovine serum albumin nanoparticles decorated with folates were developed for the targeted administration of paclitaxel.

To impart a high recognition specificity, the NP-HSAs were decorated with monoclonal antibodies against over-expressed antigens in cancer cells and tissues, such as a monoclonal antibody against αv integrins which are highly expressed in various cancer cells [17].

Similarly, it can be hypothesised to use antibodies or antibody fragments which recognise one or more surface antigens selectively expressed on cancer cells. Some of these antigens, such as Her2, are validated targets for anticancer therapies with monoclonal antibodies [18, 19]. In the case of Her2, monoclonal antibodies such as Trastuzumab or Pertuzumab can be used, which recognise different epitopes of the protein but are both capable of providing therapeutic effects in patients with antigen-expressing breast cancers. Antibodies against Her2 are particularly useful for decorating NP-HSAs, as the receptor is rapidly internalised and is able to carry antibodies, antibody fragments and antibodies conjugated with cytotoxic drugs (ADC) within the cells for a targeted and more effective therapy. Antibodies against other markers selectively expressed on cancer cells could be used for the same purpose, even if the marker is not internalised with the same efficiency. In this case, the antibody would only act as a recognition signal to guide the NP-HSAs near the cancer cell, which could subsequently internalise the NP-HSAs through the caveolae. The presence of two different recognition signals would increase specificity and the particle could be internalised through a single marker.

A further molecule of interest for these applications is the protein Cripto-1, known to be over-expressed on the surface of cancer cells of breast cancer, colon cancer, stomach cancer and other types of cancer [20]. Monoclonal antibodies which very selectively recognise human protein on the surface of cells are widely reported in the literature [20].

ADCs are a new class of highly potent biological drugs built by binding a small-molecule anticancer drug or other therapeutic agent to an antibody, using a permanent or labile linker. The antibody targets a specific antigen which is predominantly found on the target cells.

Thanks to the antibody, the ADCs selectively bind to the cancer cell receptors. The receptor-ADC complex is usually internalised by endocytosis, the linker is cleaved following degradation of the antibody and the cytotoxic drugs which induce cell death are released through various mechanisms of action, such as DNA binding or interactions with tubulin. The release from the linker can occur both near the cytotoxic substance and near the antibody, leaving the linker still anchored to the cytotoxic substance. The cytotoxic substances used in these approaches, usually small molecules, must be very powerful (≤1 nM) otherwise the concentration of antibody or fragment which carries it becomes too high and therefore uneconomical. It is therefore essential that the cytotoxic substance is released in its original form to prevent the presence of the linker or pieces of linker from altering the activity thereof. The administration of drugs by means of NP-HSAs has already shown considerable successes both preclinically and clinically. NP-HSAs have been used, for example, to trap various drugs including sorafenib, 5FU and paclitaxel [21-23]. In fact, with NP-HSAs it is possible to increase the number of drug molecules actually loaded, as these are trapped in the meshes of the nanoparticles and can be released intact over time by simple diffusion.

In this context, the use of NP-HSAs decorated with antibodies or antibody fragments is particularly useful because the NP-HSA acts as a container capable of transporting many copies of cytotoxic substance, which is guided by the antibody to the cancer cell and is released without structural alterations.

Kouchakzadeh et al., International Journal of Pharmaceutics 447 (2013) 62-69, describes HSA nanoparticles to which anti-HER2 antibodies are conjugated for use as delivery agents into tumour cells.

The need for albumin nanoparticles which are able to transport cytotoxic drugs to cancer cells, even more specifically with respect to the known nanoparticles, is therefore felt in the sector.

### SUMMARY OF THE INVENTION

The invention relates to serum albumin nanoparticles (Alb-NP) decorated with at least one biological molecule capable of recognising over-expressed or selectively expressed target receptors on the surface of cells, preferably cancer cells. The at least one biological molecule is anchored to the surface of the nanoparticles by means of a linker and a transamidation (or transglutamination) reaction mediated by the enzyme transglutaminase.

The biological molecule is selected from the group consisting of an antibody, a Fab, an scFv, a nanobody (NB) and mixtures thereof. Preferably the nanoparticles are decorated with at least two biological molecules selected from the group consisting of an antibody, a Fab, an scFv, a nanobody (NB), a peptide, in which the at least two biological molecules are different from each other.

Preferably the serum albumin used for preparing the nanoparticles is human serum albumin (HSA).

The at least one biological molecule is bound to the albumin nanoparticles through a linker with which the nanoparticles are derivatised. The derivatisation of the nanoparticles with the linker occurs by means of the formation of a covalent bond between a Z group of the linker, containing an electrophilic functionality, and the side chain of the albumin cysteines, which contains the -SH nucleophilic group (thiol).

Preferably, the covalent bond which forms between the Z group of the linker and the thiol group of the albumin cysteines is an -S- thioether bond.

The terminal part of the linker contains an X-NH₂ group which binds to a consensus sequence of a biological molecule, which contains at least one glutamine (Q; GLN). In particular, the X-NH ₂ residue of the linker binds to the glutamine of the consensus sequence by means of a transamidation (or transglutamination) reaction mediated by the enzyme transglutaminase (MTG). Alternatively, an -NH₂ residue is introduced at one end of the at least one biological molecule, for example in the form of a lysine residue inserted in a peptide sequence, and the consensus sequence containing at least one glutamine (Q; GLN) is inserted at the end of the linker in place of the X-NH ₂ group. In this case, the consensus sequence containing at least one glutamine is part of the linker. The transamidation reaction mediated by the enzyme transglutaminase occurs between the -NH₂ residue present on the at least one biological molecule and the glutamine of the consensus sequence forming part of the linker.

Preferably, the serum albumin nanoparticles decorated according to the invention are loaded with at least one cytotoxic drug, such as 5-FU, capecitabin, cytarabine, fludarabine, cladribine, paclitaxel, doxorubicin, daunorubicin, epirubicin, docetaxel, vinblastine, vincristine, vinorelbine, mercaptopurine, methotrexate, raltitrexed, etoposide, teniposide, camptothecin, irinotecan, topotecan and combinations thereof. In other words, the nanoparticles incorporate therein at least one cytotoxic drug and are decorated on the surface as indicated above.

The invention also relates to the use of serum albumin nanoparticles decorated according to the invention and loaded with a cytotoxic drug, or a pharmaceutical composition comprising the nanoparticles, to specifically treat a pathology, preferably a cancer pathology. In particular, such a pathology is selected from melanoma, breast cancer, metastatic breast cancer, glioma, glioblastoma, adenocarcinoma, intestinal cancer, pancreatic cancer, bone cancer, kidney cancer, colon cancer, stomach cancer, chronic lymphocytic leukaemia, non-small cell lung cancer, advanced and/or metastatic kidney cancer, head and neck cancer, advanced melanoma, non-Hodgkin lymphoma, metastatic melanoma, lung cancer, chronic lymphocytic leukaemia (CLL), non-Hodgkin lymphoma and age-related macular degeneration.

Also disclosed, but not part of the claimed invention, are serum albumin nanoparticles, preferably human, derivatised with the linker according to the invention, and their use for the preparation of albumin nanoparticles decorated according to the invention.

The invention also relates to a process for synthesising decorated nanoparticles which includes a step of functionalising the nanoparticles with the linker of the invention by means of the formation of a thioether bond, and conjugating the linker to at least one biological molecule by means of a transamidation or transglutamination reaction mediated by the enzyme transglutaminase.

### BRIEF DESCRIPTION OF THE FIGURES

Figure 1 shows the dose-response curve of the competitive binding to HER2 obtained with trastuzumab Fab and with the same Fab bound to human albumin particles (example 10);
Figures 2AB show the dose-response curves of the binding to Her2 receptor-expressing BT474 cells obtained with trastuzumab Fab and with the same Fab bound to human albumin particles, at 2 hours (A) and 24 hours (B) (example 11);
Figure 3A shows the dose-response curves of the binding to Her2 receptor-expressing BT474 cells and non-receptor-expressing MDA-MB-231 cells obtained with trastuzumab Fab and with the same Fab bound to human albumin particles at 2 hours (example 12);
Figures 3BC show the dose-response curves of the binding to Her2 receptor-expressing BT474 cells and non-receptor-expressing MDA-MB-231 cells obtained with trastuzumab Fab (B) and trastuzumab by incubating for 2 hours (C) (example 12);
Figure 4 shows the dose-response curves of the binding to Cripto-1-expressing NTERA cells obtained with the recombinant Fab of the anti-Cripto-1 antibody 10D1 and with the same Fab bound to human albumin particles, at 2 hours and 24 hours (example 13);
Figures 5AB show the binding of anti-Cripto-1 antibodies 1B4 and anti-Her2 trastuzumab to Her2-negative and Cripto-1-positive MDA-MB-231 cells (A) and Her2-positive and Cripto-1-positive BT474 cells (B) (example 14);
Figure 5C shows the dose-response curves of the binding to Her2-positive and Cripto-1-positive BT474 cells and Her2-negative and Cripto-1-positive MDA-MB-231 cells of bispecific NP-HSAs functionalised with 10F1 Fab and trastuzumab Fab (example 14);

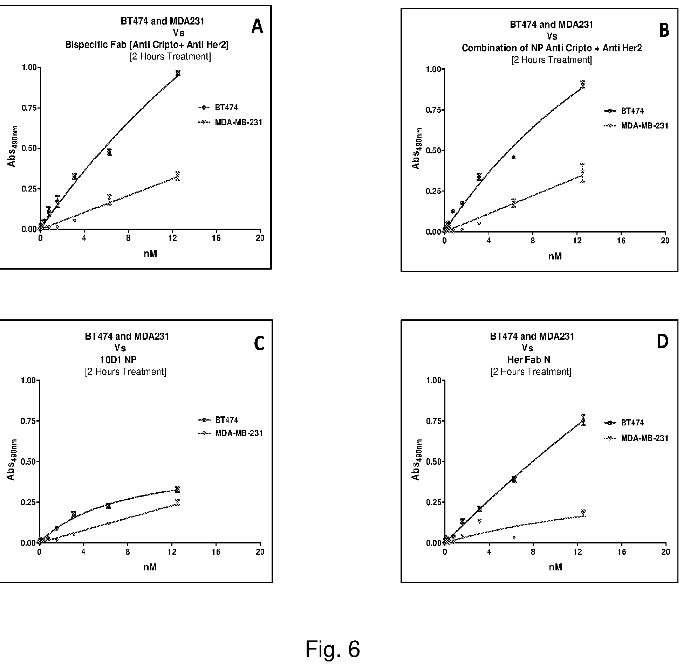

Figures 6ABCD show: the binding of bispecific NP-HSAs functionalised with trastuzumab Fab and 10D1 Fab to Her2-positive and Cripto-1-positive BT474 cells and Her2-negative and Cripto-1-positive MDA-MB-231 cells (A); the binding of the combination of NP-HSAs functionalised with trastuzumab Fab and NP-HSAs functionalised with 10D1 Fab to Her2-positive and Cripto-1-positive BT474 cells and Her2-negative and Cripto-1-positive MDA-MB-231 cells (B); the binding of NP-HSAs functionalised with 10D1 Fab to Her2-positive and Cripto-1-positive BT474 cells and Her2-negative and Cripto-1-positive MDA-MB-231 cells (C); the binding of NP-HSAs functionalised with trastuzumab Fab to Her2-positive and Cripto-1-positive BT474 cells and Her2-negative and Cripto-1-positive MDA-MB-231 cells (D) (example 15).

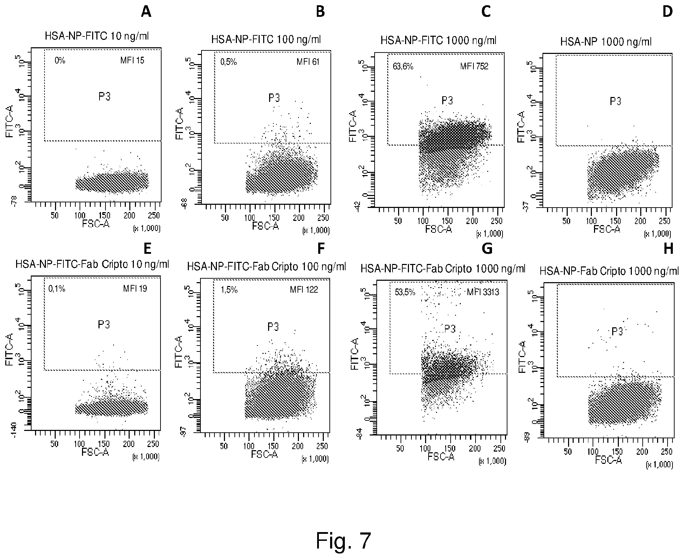

Figures 7A-H show: from A to C the binding of FITC-NP-HSA to Cripto-1-expressing NTERA2 cells at concentrations of 10, 100, 1000 ng/mL, respectively; in D the binding of NP-HSAs not functionalised with FITC to the same cells at 1000 ng/mL; from E to G the binding of FITC-NP-HSA-Fab to Cripto-1-expressing NTERA2 cells at concentrations of 10, 100, 1000 ng/mL; H shows the binding of the NP-HSA-Fabs not functionalised with FITC to the same cells.

### DETAILED DESCRIPTION OF THE INVENTION

All the amino acids indicated in the present description are preferably in the L configuration. In a first aspect, the invention relates to serum albumin nanoparticles (Alb-NP) decorated with at least one decoration chain comprising:
- a linker, bound to the nanoparticles by means of an -S- thioether bond, and
- at least one biological molecule
as defined in present claim 1.

The at least one biological molecule is bound to the linker through an amide bond formed by means of a transamidation (or transglutamination) reaction, mediated by the enzyme transglutaminase, between:
(i) the -NH₂ residue of an X group of a linker and the CO-NH₂ residue of a glutamine comprised in a peptide consensus sequence inserted in the at least one biological molecule; or
(ii) the -NH₂ residue of a lysine comprised in a peptide sequence inserted in the at least one biological molecule and the -CO-NH₂ residue of a glutamine inserted in a consensus sequence which is part of the linker.

In case (i), the linker is derived from the precursor of formula (IV): 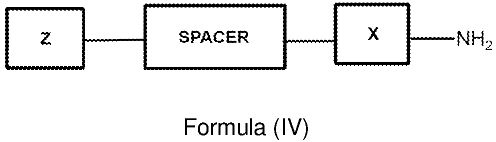 wherein,
the -NH₂ residue of the X group forms an amide bond with the CO-NH₂ residue of a glutamine (Q; GLN) of a consensus sequence, contained in the at least one biological molecule, in which the consensus sequence has the following formula (VI):

AA₁-AA₂-Q-AA₃-AA₄ Formula (VI)

in which:
AA₁ is leucine (L; Leu) or is absent;
AA₂ is leucine (L; Leu) or is threonine (T; Thr);
Q is glutamine with the formula -CO-(CH₂)₂-CH-(NH)-CO-;
AA₃ is serine (S; Ser) or is glycine (G; Gly);
AA₄ is proline (P; Pro), alanine (A; Ala) or is absent.

Preferably, AA₁ and AA₄ are not simultaneously absent.

The consensus sequence is preferably selected from: LQSP, TQGA, LLQG.

In case (ii), the linker is derived from a precursor comprising the consensus sequence of formula (VI) which is linked to the spacer by means of the amino acid AA₁ or AA₂, according to the following formula (VII):

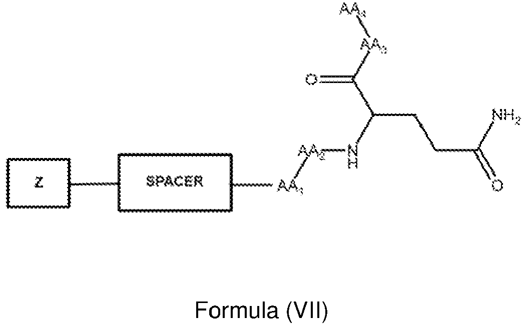

In both cases, the Z group is bound to the surface of the nanoparticles by means of an -S-thioether bond.

The serum albumin nanoparticles decorated according to the invention have the formula (I) or (II):

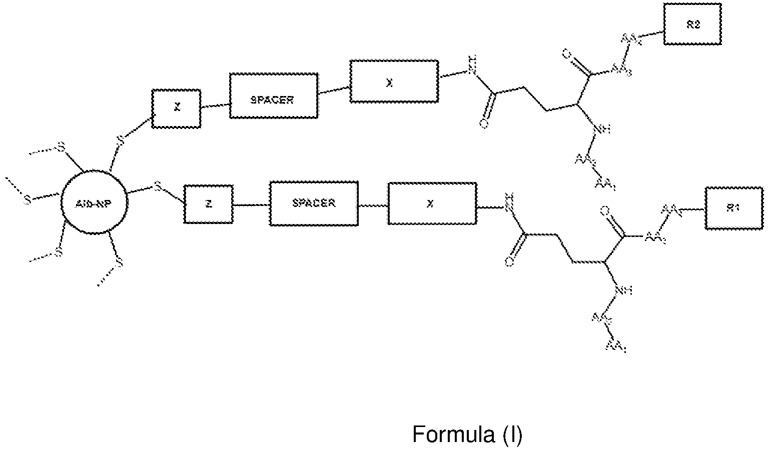

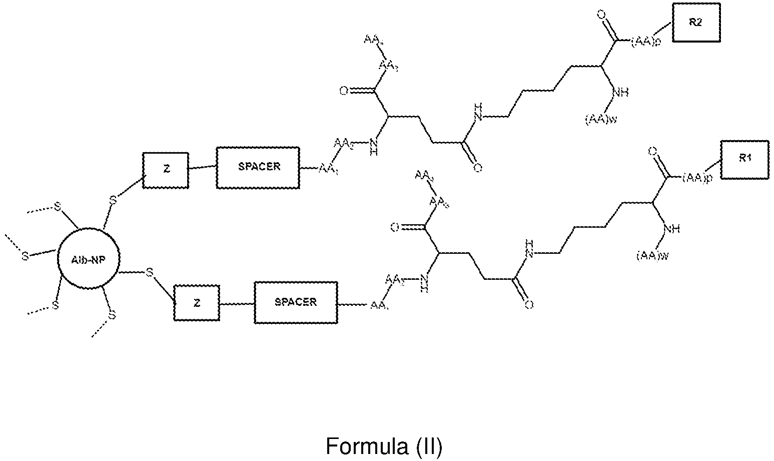

In formula (I), the following fragments can be identified:

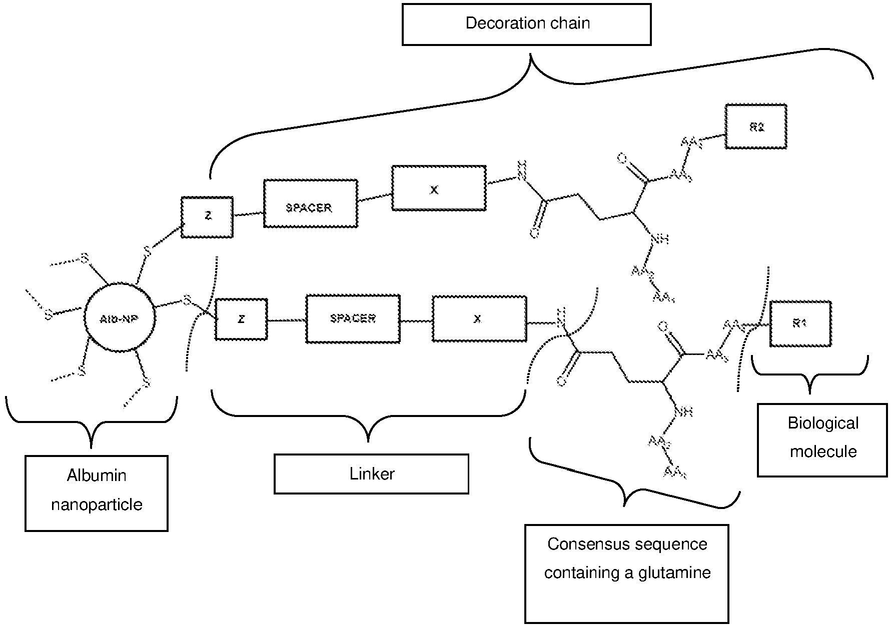

In formula (II), the following fragments can be identified:

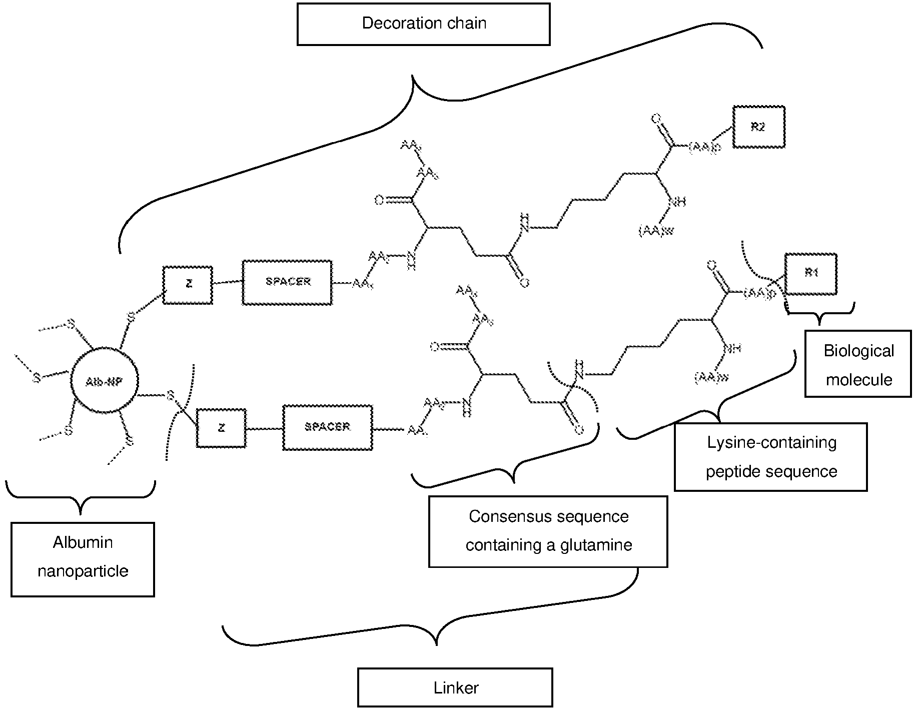

The transamidation reaction mediated by the enzyme transglutaminase is schematically shown below (diagram I):

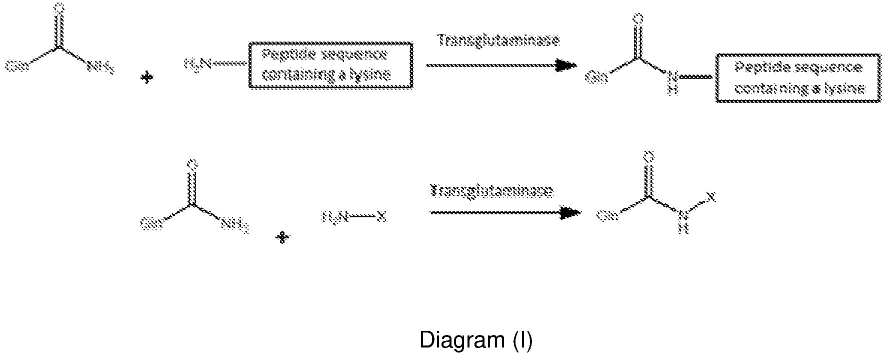

With reference to formulas (I) and (II), the serum albumin nanoparticles, represented by the symbol Alb-NP, are bovine serum albumin nanoparticles (NP-BSA) or human serum albumin nanoparticles (NP-HSA). Preferably, they are NP-HSAs.

The albumin nanoparticles have the -SH thiol groups of the side chain of the cysteine residues, preferably of the albumin cysteine 34, bound, by means of a thioether bond (-S-), to the Z group of the linker.

The precursor of the albumin nanoparticles (Alb-NP) is represented by the following formula (III):

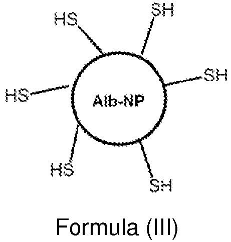

The symbol in formula (I) indicates the presence of a plurality of decoration chains bound to the nanoparticles by means of thioether bond. In formula (I), only a decoration chain of the nanoparticles is fully represented for the simplicity of the depiction.

The nanoparticles have an average diameter (or Z-average size) of about 100-500 nm, preferably 100-400 nm or 300-400 nm, measured with the Dynamic Light Scattering (DLS) technique.

The nanoparticles have a polydispersity index (PDI) comprised between 0.02 and 0.05.

The albumin nanoparticles are preferably loaded with at least one cytotoxic drug, such as 5-FU, capecitabin, cytarabine, fludarabine, cladribine, paclitaxel, doxorubicin, daunorubicin, epirubicin, docetaxel, vinblastine, vincristine, vinorelbine, mercaptopurine, methotrexate, raltitrexed, etoposide, teniposide, camptothecin, irinotecan, topotecan and combinations thereof.

The term "loaded" means that the cytotoxic drug is incorporated within the nanoparticles during the preparation thereof and before their subsequent decoration with the linker and the biological molecule.

The albumin nanoparticles are prepared by methods known in the art, for example by the method of desolvation and subsequent stabilisation with crosslinker which may be, for example, glutaraldehyde or diazirine. Another method for preparing albumin nanoparticles is the high-pressure homogenisation method.

Other known preparation methods are emulsification, thermal gelation, drying and self-assembly.

If the nanoparticles are loaded with a cytotoxic drug, the latter is dissolved or suspended in the starting albumin solution and then incorporated within the nanoparticles during their formation by the methods described above.

The **linker** used to decorate the nanoparticle of the invention, as indicated in Formula (I), comprises a Z group, a spacer and an X-NH- group. The linker is derived from the precursor of formula (IV): 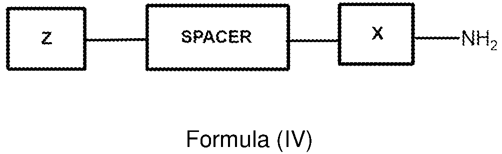 wherein,
the Z group is derived from a functional group containing an electrophilic group capable of reacting with the -SH group of the albumin cysteines, preferably cysteine 34, and forming a thioether bond in a pH range comprised between 4 and 9.

According to the invention, the Z group is introduced in the linker starting from the derivatives listed in the following table 1:

**Table 1**

| | **Z Group** | **Electrophilic group** |
|---|---|---|
| 1 | 2-bromoacetic acid | Bromine |
| 2 | 3-bromopropanoic acid | Bromine |
| 3 | 3-chloropropanoic acid | Bromine |
| 4 | 4-bromobutyric acid | Bromine |
| 5 | 5-chlorobutyric acid | Chlorine |
| 6 | 5-bromopentanoic acid | Chlorine |
| 7 | 5-chloropentanoic acid | Chlorine |
| 8 | 4-bromomethyl benzoic acid | Bromine |
| 9 | 4-chloromethyl benzoic acid | Chlorine |
| 10 | 2-maleimidoacetic acid | maleimide |
| 11 | 3-malemidopropionic acid | maleimide |
| 12 | 4-maleimidobutyric acid | maleimide |
| 13 | 5-maleimidopentanoic acid | maleimide |
| 14 | 6-maleimidohexanoic acid | maleimide |
| 15 | 3-maleimidobenzoic acid | maleimide |
| 16 | 4-maleimidobenzoic acid | maleimide |
| 17 | 4-(2-N-Maleimido)methyl benzoic acid | maleimide |
| 18 | 1-bromoacetic acid | bromine |
| 19 | 1-chloroacetic acid | chlorine |

Preferably Z derives from a functional group selected from: 1-bromoacetic acid, 1-chloroacetic acid and 6-maleimidohexanoic acid.

The spacer is an inert molecule under both physiological and extreme pH conditions (e.g., pH < 3 or pH > 9) which acts as a spacer between the X-NH ₂ group and the subsequent Z group. The spacer is a flexible molecule which in an extended configuration can reach the size of at least 10 Angstroms.

According to the invention, the spacer is selected from:
- -NH-(CH₂-O)ₙ-CH₂-CO-, with n ranging between 2 and 10, preferably 2, 3, 4 and 5;
- -NH-(CH₂-CH₂-O)ₙ-CH₂-CO-, with n ranging between 2 and 10, preferably 2, 3, 4 and 5;

or the spacer is a Yₘ group,
where Y is selected from:
   - an NH-(CH₂)ₙ-CO- amino acid, with n ranging between 3 and 10, preferably between 3 and 5, more preferably with n = 3, 4 or 5, i.e., aminocaproic acid ε, aminopentanoic acid δ and aminobutyric acid γ, respectively;
   - glycine, alanine;
   - and combinations thereof;
m is a number comprised between 1 and 5.

Therefore, Yₘ denotes a single amino acid or a polypeptide consisting of the indicated amino acids and of dimensions comprised between a dipeptide and a pentapeptide.

The -CO- group of the spacer forms an amide bond with the -NH- group of the subsequent X unit and the -NH- group of the spacer forms an amide bond with the acid group of the precursor of Z.

Preferably, the spacer is -NH-(CH₂-CH₂-O)ₙ-CH₂-CO-, with n ranging between 2 and 5 or it is glycine.

The X-NH ₂ group of the formula (I) comprises a primary amine group bound to an X group comprising an alkyl or methoxyalkyl chain or other non-reactive flexible chain under normal physiological conditions or under extreme pH conditions (e.g., pH < 3 or pH > 9). Preferably, X is an alkyl chain containing at least 3 methylene groups or a chain containing at least 2 methoxyethylene groups.

According to the invention, the X-NH₂ group is selected from:
- -NH-(CH₂)ₙ-NH₂ with n ranging between 3 and 10, preferably 3, 4 and 5;
- -NH-(O-CH₂)ₙ-NH₂, with n ranging between 2 and 10, preferably 2, 3, 4 and 5;
- -NH-(O-CH₂-CH₂)ₙ-NH₂, with n ranging between 2 and 10, preferably 2, 3, 4 and 5;
- L-lysineamino acid;
- L-ornithine amino acid;
- C-terminal amidated L-lysine amino acid;
- C-terminal amidated L-ornithine amino acid;

The X-NH₂ group is linked to the spacer group through an amide bond formed between an amine group of X and a carboxyl group coming from the spacer.

If the X-NH₂ group is an amino acid selected from L-lysine, L-ornithine, L-lysinamide and L-ornithinamide, the amine group which forms the amide bond is the alpha-amine group.

Preferably, the X-NH₂ group is selected from L-lysine amino acid and C-terminal amidated L-lysine amino acid.

In one embodiment, the Z group is selected from: 1-bromoacetic acid, 1-chloroacetic acid and 6-maleimidohexanoic acid; the spacer is -NH-(CH₂-CH₂-O)ₙ-CH₂-CO-, with n ranging between 2 and 5, or it is glycine; the X-NH₂ group is L-lysine amino acid or C-terminal amidated L-lysine amino acid.

In one embodiment, the albumin nanoparticles, preferably NP-HSA, are derivatised with a linker comprising a Z group selected from: 1-bromoacetic acid, 1-chloroacetic acid and 6-maleimidohexanoic acid; a spacer -NH-(CH₂-CH₂-O)ₙ-CH₂-CO-, with n ranging between 2 and 5 or glycine; an X-NH₂ group selected from L-lysine amino acid and C-terminal amidated L-lysine amino acid.

Preferably, the derivatised nanoparticles are loaded with at least one cytotoxic drug selected from: 5-FU, sorafenib, paclitaxel, doxorubicin and combinations thereof.

An example of linker precursor according to formula (I) is Br-CH₂-CO-O2Oc-K-NH₂ prepared according to example 8, of formula (V): 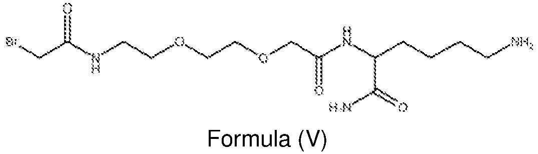 Formula (V) is shown below with the indication of the various residues according to formula (I):

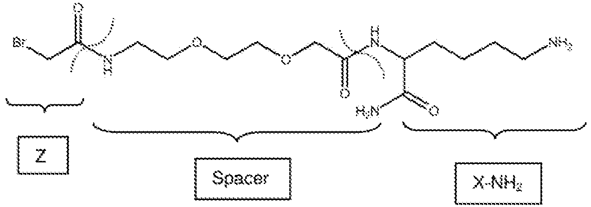

Another example of a linker precursor is Mal-Gly-Lys-CONH₂ prepared according to example 9, of formula (VI):

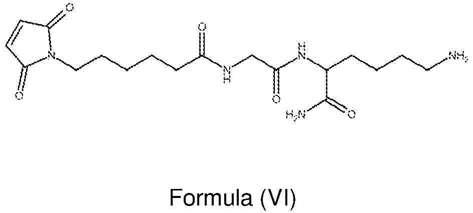

Formula (VI) is shown below with the indication of the various residues according to formula (I):

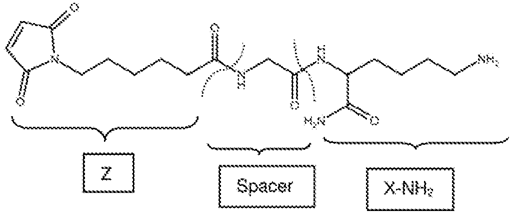

In the case of formula (II), the linker is derived from the precursor of formula (VII): 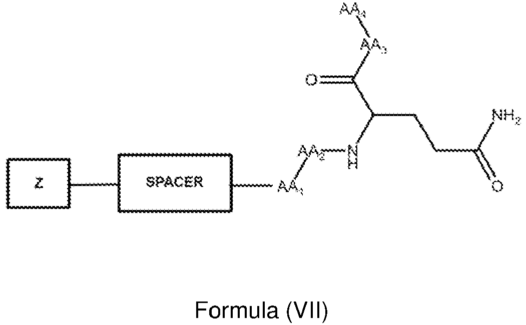 wherein
Z and spacer are defined as above for formula (IV), while the X-NH₂ group is replaced by a consensus peptide sequence containing at least one glutamine (Q; GLN) of formula (VIII):

AA₁-AA₂-Q-AA₃-AA₄ Formula (VIII)

wherein:
AA₁ is leucine (L; Leu) or is absent;
AA₂ is leucine (L; Leu) or is threonine (T; Thr);
Q is glutamine;
AA₃ is serine (S; Ser) or is glycine (G; Gly);
AA₄ is proline (P; Pro), alanine (A; Ala) or is absent.

Preferably, AA₁ and AA₄ are not simultaneously absent.

The consensus sequence is preferably selected from: LQSP, TQGA, LLQG.

The consensus sequence is bound to the spacer by an amide bond between the -CO-terminal of the spacer and an -NH- group of the amino acid AA₁ (if present) or AA₂.

The reaction of the linkers with the albumin cysteines present on the surface of the nanoparticles occurs in buffered aqueous solutions at a pH comprised between 4 and 9, preferably ≥ 8. The conjugation reactions are generally complete in a time interval not exceeding 16h at room temperature (25 °C).

The number of linker molecules which can be bound on the nanoparticles depends on the number of thiol groups exposed on the surface. For example, an approximate calculation based on NP-HSAs with an average diameter of 100 nm and HSA molecules having an approximate diameter comprised between 7 and 10 nm allows to estimate an amount of reactive thiol groups comprised between 2 and 3 nmoles for each mg of NP-HSA prepared with the methods described in the literature.

The albumin nanoparticles can be treated with the Traut reagent, 2-iminothiolane, so as to increase the number of free thiols on the nanoparticles and thus increase the density of the linkers which can be introduced onto their surface.

The linkers according to the present invention contain a Z group, a spacer and an X group or a consensus sequence containing a glutamine which are connected by means of amide bonds. The advantage of having a linker with amide bonds distributed throughout the chain lies in its high stability even under extreme chemical-physical conditions (for example pH comprised between 1 and 9), in the ability to confer solubility to the resulting decorated nanoparticles, in the ease of linker synthesis both with chemical synthesis methods and with biological methods, for example by means of enzymes.

The derivatisation of the nanoparticles with the linkers allows to obtain nanoparticles functionalised with amine groups, derived from the linker, which can react with at least one biological molecule bearing a consensus sequence comprising a glutamine (linker of formula (IV)), or a peptide sequence comprising a lysine (linker of formula (Vll)). The reaction between the amine group of the linker in the case of the linker of formula (IV), or the amine group of the peptide sequence included in the biological molecule (in the case of the linker of formula (VII)), and the glutamine of the consensus sequence is a transamidation (or transglutamination) reaction mediated by the enzyme transglutaminase. Preferably, the transglutaminase is bacterial transglutaminase, referred to as MTG.

This reaction allows to covalently bind to the surface of albumin nanoparticles bearing amine groups introduced by derivatisation with the linkers of the invention, in an oriented and site-specific manner, through an isopeptide amide bond, polypeptide chains of biological molecules.

It is important to note that the albumin of the nanoparticles, while possessing multiple lysines and glutamines as well as amine groups derived from the N-terminals of the albumin molecules, does not undergo transglutamination reactions mediated by transglutaminase since such glutamines and lysines of the albumin molecule are not reactive towards the enzyme transglutaminase. Some biomolecules are known to contain transglutaminase-reactive glutamine residues. Such molecules can similarly be used in these applications.

In the case of the albumin nanoparticles of formula (I) and the linker of formula (IV), the consensus sequence contained in the biological molecule is the peptide sequence containing at least one glutamine (Q; GLN) of formula (VIII) as set out above.

In the case of albumin nanoparticles of formula (II), the peptide sequence containing a lysine (K; Lys) has the following formula (IX):

(AA)_{w}-K-(AA)ₚ Formula (IX)

wherein
w and p are whole numbers comprised between 0 and 8, preferably between 1 and 5, with the condition that w and p are never simultaneously equal to 0;
AA indicates an amino acid selected from: alanine (A; Ala), tyrosine (Y; Tyr), phenylalanine (F; Phe), glycine (G; Gly), tryptophan (W; Trp) and serine (S; Ser);
K is a lysine (Lys).

Preferably, w is equal to 0 and p is equal to 1-3; more preferably w is equal to 0 and p is equal to 3.

The peptide sequence is preferably selected from:
KAYA, KGYA, KSYA, KAFA, KGFA, KSFA, KAWA, KGWA, KSWA, KAYG, KGYG, KSYG, KAFG, KGFG, KSFG, KAWG, KGWG, KSWG, KAYS, KGYS, KSYS, KAFS, KGFS, KSFS, KAWS, KGWS, KSWS.

In one embodiment albumin nanoparticles, preferably NP-HSAs, are derivatised with a linker comprising a Z group selected from: 1-bromoacetic acid, 1-chloroacetic acid and 6-maleimidohexanoic acid; a spacer -NH-(O-CH₂-CH₂)ₙ-CO-, with n ranging between 3 and 5 or glycine; an X-NH₂ group selected from L-lysine amino acid, C-terminal amidated L-lysine amino acid, wherein the terminal X-NH₂ group of the linker is bound by means of amide bond to the glutamine in a consensus sequence selected from: LQSP, TQGA, LLQG included in a biological molecule.

Preferably, such nanoparticles are loaded with at least one cytotoxic drug selected from: 5-FU, capecitabin, cytarabine, fludarabine, cladribine, paclitaxel, doxorubicin, daunorubicin, epirubicin, docetaxel, vinblastine, vincristine, vinorelbine, mercaptopurine, methotrexate, raltitrexed, etoposide, teniposide, camptothecin, irinotecan, topotecan and combinations thereof.

The R1 and R2 groups of formula (I) represent a biological molecule selected from: an antibody, a Fab, an scFv, a nanobody (NB) and mixtures thereof.

R1 and R2 can be identical or different from one another.

The antibody is preferably a monoclonal antibody, preferably selected from: Trastuzumab or Pertuzumab, monoclonal antibodies against HER2, surface protein selectively expressed by the cancer cells of breast cancers; Trastuzumab and Pertuzumab recognise different epitopes of the protein HER2; Cetuximab, anti-EGFR antibody; anti-Cripto-1 monoclonal antibody, e.g., anti-Cripto-1 antibody 1B4 or anti-Cripto-1 antibody 10D1 recently reported in the literature [24], or other antibodies against antigens selectively expressed on the surface of cancer cells.

Fab is preferably a recombinant Fab generated from monoclonal antibodies which bind receptors of biological interest such as antibodies against cancer antigens selectively expressed on the surface of cancer cells, such as the recombinant Fab of Trastuzumab (prepared as described in Selis et al [25]) or Pertuzumab or other Fabs obtained by mutating the polypeptide sequences of known monoclonal antibodies such as the anti-Cripto-1 antibodies 1B4 and 10D1. Such Fabs can be recombinantly generated as described in Selis et al., [25]so as to contain a consensus sequence at the C-terminal of the heavy chain, e.g., the TQGA sequence, and be enzymatically conjugated to the linker present on the surface of the albumin nanoparticles.

ScFv is a functional fragment of an antibody selected from: Trastuzumab, Pertuzumab, Cetuximab, an anti-Cripto-1 monoclonal antibody, e.g., anti-Cripto-1 antibody 1B4 or anti-Cripto-1 antibody 10D1, antibodies against cancer antigens selectively expressed on the surface of cancer cells.

The nanobodies (NBs) are selected from NBs against VEGFR2, such as 3VGR19 NB [26], against Her2, such as 5F7GGC NB [27], or against EGFR, such as EGa1 [27].

With regard to formula (I), the method for introducing a consensus sequence into a biological molecule is known in the art, for example from [25].

The consensus sequence is introduced into the biological molecule in a position distant from the active regions of the molecule itself.

The advantage of using consensus sequences introduced into biological molecules in a position distant from the active regions of the molecule itself consists in the ability to bind the biological molecules to the linker of formula (IV) present on the nanoparticles of the invention so that they are all positioned with the active regions, for example the complementary-determining regions (CDRS), oriented outwards of the nanoparticle. The outwards orientation of the active regions of the biological molecules promotes the recognition and interaction with the receptors present on the target cells, for example present on cancer cells.

Similarly, in the case of formula (II), the method for introducing a peptide sequence containing a lysine onto the biological molecule is known in the art.

The peptide sequence is introduced into the biological molecule in a position distant from the active regions of the molecule itself.

The advantage of using a peptide sequence introduced into biological molecules in a position distant from the active regions of the molecule itself consists in the ability to bind the biological molecules to the linker of formula (VII) present on the nanoparticles of the invention so that they are all positioned with the active regions, for example the complementary-determining regions (CDRS), oriented outwards of the nanoparticle. The outwards orientation of the active regions of the biological molecules promotes the recognition and interaction with the receptors present on the target cells, for example present on cancer cells.

With the method of anchoring biological molecules to the linkers described in the present invention, it is possible to anchor between 2 µg and 80 µg of biological molecule per mg of albumin nanoparticles, preferably between 5 and 50 µg.

For example, in the case where the biological molecule is a Fab, which has a molecular weight of about 50 kDa, it is possible to anchor about 200 pmoles of biological molecule per mg of nanoparticles; in the case of scFv, which has a molecular weight of about 25 kDa, the density becomes 400 pmoles/mg of nanoparticles; in the case of an NB, which has a molecular weight of about 12.5 kDa, the density becomes 800 pmoles/mg of nanoparticles. The biological molecule density per unit weight of the nanoparticles is comprised between 6% and 40%, preferably between 0.2% and 8% for Fabs, between 0.4 and 16% for scFvs and between 1% and 33% for NB.

In one embodiment, the albumin nanoparticles, preferably NP-HSAs, are derivatised with a linker comprising a Z group selected from: 1-bromoacetic acid, 1-chloroacetic acid and 6-maleimidohexanoic acid; a spacer -NH-(CH₂-CH₂-O)ₙ-CH₂-CO-, with n ranging between 3 and 5 or glycine; an X-NH₂ group selected from: L-lysine amino acid and C-terminal amidated L-lysine amino acid, in which the terminal group X-NH₂ is bound by means of amide bond to the glutamine of a consensus sequence selected from: LQSP, TQGA, LLQG included in a biological molecule, in which the biological molecule is selected from: an antibody, a Fab, an scFv, a nanobody (NB) and mixtures thereof.

Preferably, the nanoparticles are loaded with a cytotoxic drug selected from: 5-FU, capecitabin, cytarabine, fludarabine, cladribine, paclitaxel, doxorubicin, daunorubicin, epirubicin, docetaxel, vinblastine, vincristine, vinorelbine, mercaptopurine, methotrexate, raltitrexed, etoposide, teniposide, camptothecin, irinotecan, topotecan and combinations thereof.

In a particularly preferred embodiment, the groups R1 and R2 are different from each other.

For example, R1 and R2 are two different types of Fab, which can be referred to as Fab1 and Fab2, two different types of scFv, which can be referred to as scFv1 and scFv2, two different types of NB, which can be referred to as NB1 and NB2, two different types of antibody which can be referred to as Ab1 and Ab2 or two different peptides. Alternatively, R1 and R2 are mixed combinations of biological molecules, for example Fab1 and scFv1, Fab1 and NB2, or scFv1 and NB2, or Ab1 and NB1.

The combinations are biological molecules are selected from the biological molecules listed above.

Preferably, R1 is the recombinant anti-Cripto-1 Fab 10D1 and R2 is the anti-HER2 Fab, or R1 is Trastuzumab and R2 Cetuximab; R1 is Trastuzumab and R2 Rituximab; R1 Trastuzumab and R2 Ipilimumab; R1 Trastuzumab and R2 Alemtuzumab; R1 Trastuzumab and R2 Nivolumab; R1 Trastuzumab and R2 Pembrolizumab; R1 Trastuzumab and R2 Panitumumab; R1 Trastuzumab and R2 Ibritumab tiuxetan; R1 Trastuzumab and R2 Tositumomab; R1 Trastuzumab and R2 Bevacizumab; R1 Trastuzumab and R2 Ofatumumab; R1 is the recombinant anti-Cripto-1 Fab 10D1 and R2 Cetuximab; R1 is the recombinant anti-Cripto-1 Fab 10D1 and R2 Rituximab; R1 is the recombinant anti-Cripto-1 Fab 10D1 and R2 Ipilimumab; R1 is the recombinant anti-Cripto-1 Fab 10D1 and R2 Alemtuzumab; R1 is the recombinant anti-Cripto-1 Fab 10D1 and R2 Nivolumab; R1 is the recombinant anti-Cripto-1 Fab 10D1 and R2 Pembrolizumab; R1 is the recombinant anti-Cripto-1 Fab 10D1 and R2 Panitumumab; R1 is the recombinant anti-Cripto-1 Fab 10D1 and R2 Ibritumomab tiuxetan; R1 is the recombinant anti-Cripto-1 Fab 10D1 and R2 Tositumomab; R1 is the recombinant anti-Cripto-1 Fab 10D1 and R2 Bevacizumab; R1 is the recombinant anti-Cripto-1 Fab 10D1 and R2 Ofatumumab.

The decoration of the nanoparticle with at least two biological molecules is carried out using equimolar solutions of the molecules.

For example, nanoparticles carrying on the surface at least 10 µg total of Fab1 and Fab2 mixture can be prepared.

This aspect of the invention is particularly relevant because it allows the nanoparticle thus obtained to recognise at least two different receptors on the surface of the cancer cells which over-express them with consequent increase in the delivery specificity of the anti-cancer drug.

In one embodiment, the albumin nanoparticles, preferably NP-HSAs, are derivatised with a linker comprising a Z group selected from: 1-bromoacetic acid, 1-chloroacetic acid and 6-maleimidohexanoic acid; a spacer -NH-(CH₂-CH₂-O)ₙ-CH₂-CO-, with n ranging between 3 and 5 or glycine; an X-NH₂ group selected from: L-lysine amino acid and C-terminal amidated L-lysine amino acid, in which the terminal group X-NH₂ is bound by means of amide bond to the glutamine of a consensus sequence selected from: LQSP, TQGA, LLQG included in a biological molecule. The biological molecule is represented by R1 and R2, in formula (I), in which R1 and R2 are the same or different from each other.

Preferably, the nanoparticles are loaded with a cytotoxic drug selected from: 5-FU, capecitabin, cytarabine, fludarabine, cladribine, paclitaxel, doxorubicin, daunorubicin, epirubicin, docetaxel, vinblastine, vincristine, vinorelbine, mercaptopurine, methotrexate, raltitrexed, etoposide, teniposide, camptothecin, irinotecan, topotecan and combinations thereof.

In a preferred embodiment, the nanoparticles are NP-HSAs derivatised with the linker of formula (IV) or formula (VII) and conjugated to the recombinant anti-HER2 Fab and/or the recombinant anti-Cripto Fab 10D1, by a consensus sequence comprising at least one glutamine, preferably of formula (VIII).

In one embodiment of the invention, the albumin nanoparticles, loaded or not loaded with a cytotoxic drug, can be derivatised with a fluorescent dye which binds to the side chains of the lysines of the albumin molecule and subsequently treated with the linker of the invention and then subjected to bioconjugation reactions with transglutaminase to anchor the biological molecule through a consensus sequence. A suitable fluorescent dye for the purpose is, for example, fluorescein isothiocyanate (FITC) which is capable of reacting with the primary amine groups of the lysines of the albumin molecule, in the absence of aggressive or harmful chemical reagents for the albumin molecules.

Preferably, the decorated albumin nanoparticles according to the invention are selected from the following formulas: 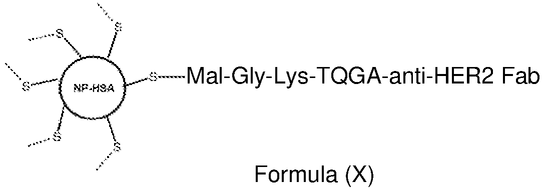 the preparation of which is described in example 4; 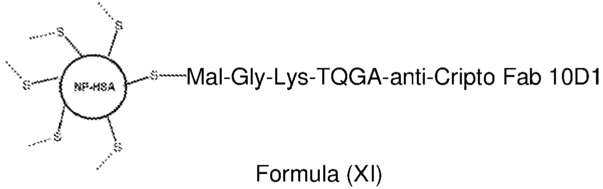 the preparation of which is described in example 5; 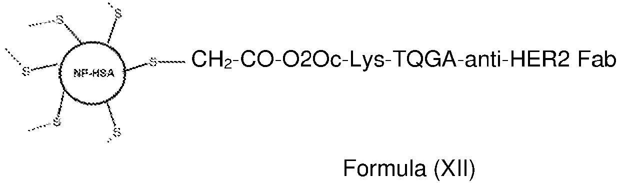 the preparation of which is described in example 6; 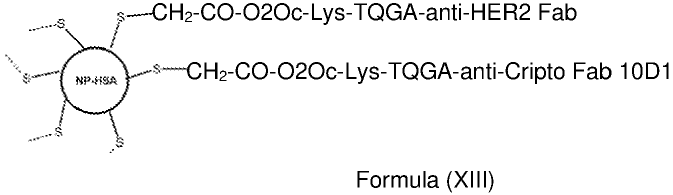 the preparation of which is described in example 7; 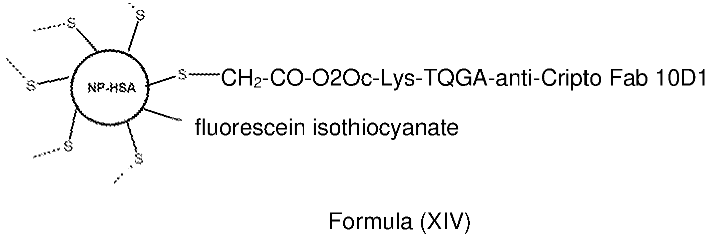 the synthesis of which is described in example 16.

In a further aspect, the present disclosure relates to a pharmaceutical composition comprising albumin nanoparticles, preferably NP-HSAs, decorated according to the invention and loaded with at least one cytotoxic drug, and pharmaceutically acceptable excipients. In a further aspect, the present disclosure relates to albumin nanoparticles decorated according to the invention and loaded with a cytotoxic drug, or a pharmaceutical composition which contains them, for use in the treatment of a cancer pathology, preferably selected from: melanoma, breast cancer, metastatic breast cancer, glioma, glioblastoma, adenocarcinoma, intestinal cancer, pancreatic cancer, bone cancer, kidney cancer, colon cancer, stomach cancer, chronic lymphocytic leukaemia, non-small cell lung cancer, advanced and/or metastatic kidney cancer, head and neck cancer, advanced melanoma, non-Hodgkin lymphoma, metastatic melanoma, lung cancer, chronic lymphocytic leukaemia (CLL), non-Hodgkin lymphoma and age-related macular degeneration.

Preferably, the albumin nanoparticles are NP-HSAs and are decorated with at least two different types of biological molecules. In other words, R1 and R2 of the formula (I) are different from each other.

Also disclosed, but not part of the claimed invention, is a method for treating a cancer pathology, preferably selected from: melanoma, breast cancer, metastatic breast cancer, glioma, glioblastoma, adenocarcinoma, intestinal cancer, pancreatic cancer, bone cancer, kidney cancer, colon cancer, stomach cancer, chronic lymphocytic leukaemia, non-small cell lung cancer, advanced and/or metastatic kidney cancer, head and neck cancer, advanced melanoma, non-Hodgkin lymphoma, metastatic melanoma, lung cancer, chronic lymphocytic leukaemia (CLL), non-Hodgkin lymphoma and age-related macular degeneration, which comprises the administration of an effective amount of nanoparticles decorated according to the invention and loaded with a cytotoxic drug, or a composition which contains them, to a patient in need. Preferably, the albumin nanoparticles are NP-HSAs and are decorated with at least two different types of biological molecules. In other words, R1 and R2 of the formula (I) are different from each other.

### Example 1.

### Preparation of NP-HSA and NP-HSA-5FU by desolvation and stabilisation method using glutaraldehyde.

For the preparation of the HSA nanoparticles, the desolvation method [29,30] was used as described below:
100 mg of human albumin (HSA fatty acid free, Sigma, MO, USA) was solubilised in 2 mL of deionised water and brought to pH 8.6 by the addition of 0.1 M NaOH. Under constant stirring, 8 mL of 99.8% ethanol (Sigma, MO, USA) was added to the solution, drop by drop and at a flow of 1 mL/min. For the preparation of the nanoparticles loaded with the 5-FU, 100 mg of HSA and 4 mg of 5FU (Sigma Aldrich, MO, USA) were solubilised in 2.2 mL of purified water and brought to a pH of 8.6. The solution was kept in incubation and under constant stirring for two hours before proceeding with the desolvation with ethanol. After the desolvation process, the NP-HSAs, always under constant magnetic stirring, were stabilised by the addition of 38 uL of glutaraldehyde, Grade II, at 25% in H₂O (MO, Sigma) and left stirring for 24 hours. The NP-HSAs were then purified with three wash cycles with H₂O by centrifugation so as to remove the ethanol and glutaraldehyde. Finally, they were re-dispersed in PBS and stored at +4°C to be characterised by size (diameter in nm), polydispersity (polydispersity index, PDI) and zeta potential (in mV) using a Malvern Zetasizer Ultra Dynamic Light Scattering (DLS) system (Malvern Instruments, Worcestershire, UK). For the size measurements and PDI determination, the nanoparticles were diluted in PBS up to a concentration of 0.1 mg/mL NP-HSA and analysed at +25°C, with multi-angle scattering, and using micro cuvettes (UV-Cuvette, Brand^{®}, Wertheim, Germany). For the zeta potential measurements to determine surface load, the NP-HSAs were diluted in ultra-pure H₂O up to a concentration of 0.1 mg/mL NPs and analysed at +25°C by disposable capillary cells DTS1070 (Malvern Instruments, Worcestershire, UK). All the size and Zeta Potential measurements were done in triplicate and represented as mean ± standard deviations.

The NP-HSAs were analysed by DLS, from which the mean diameter was found to be 130 nm with a PDI of 0.04.

### Example 2.

### Preparation of NP-HSA by desolvation and stabilisation method using diazirine.

For the preparation of the NP-HSAs, the desolvation method [29,30] was used as described below:
100 mg of HSA fatty acid free albumin (Sigma, MO, USA) was solubilised in 2 mL of purified water and brought to pH 8.6 by the addition of 0.1 M NaOH. Under constant stirring, 8 mL of 99.8% ethanol (Sigma, MO, USA) was added to the solution, drop by drop and at a flow of 1 mL/min. After the desolvation process, the nanoparticles, always under constant magnetic stirring, were stabilised by the addition of a photo-crosslinker: NHS-Diazirine (SDA) (Sigma, MO, USA). In particular, 1.68 mg of NHS-Diazirine were solubilised in 0.5 ml of anhydrous DMSO and added to the suspension so as to reach a molar ratio HSA/NHS-diazirine of 1:5 and left in incubation for 3 hours. The suspension was then dialysed by means of cellulose membrane with MWCO 6000 Da for 16 hours and finally, once the excess NHS-diazirine was removed, the suspension was exposed to UV (360 nm) for 10 minutes and under constant stirring so as to photo-stabilise the NP-HSAs. The NP-HSAs were then purified with three wash cycles with H₂O by centrifugation, finally re-dispersed in PBS and stored at +4°C to be characterised by size (nm), PDI and zeta potential (mV) by means of Malvern Zetasizer Ultra (Malvern Instruments, Worcestershire, UK). For the size determination and PDI determination, the NP-HSAs were diluted in PBS up to a concentration of 0.1 mg/mL NPs and analysed at +25°C, with multi-angle scattering, and using micro cuvettes (UV-Cuvette, Brand^{®}, Wertheim, Germany). For the zeta potential measurements to determine surface load, the NP-HSAs were diluted in ultra-pure H₂O up to a concentration of 0.1 mg/mL NPs and analysed at +25°C by disposable capillary cells DTS1070 (Malvern Instruments, Worcestershire, UK).

The NP-HSAs were analysed by DLS, from which the mean diameter was found to be 340 nm with a PDI of 0.04.

### Example 3.

### Preparation of NP-HSA by high-pressure homogenisation

For the preparation of the NP-HSAs by high pressure homogenisation, 100 mg of HSA fatty acid free albumin (Sigma, MO, USA) is solubilised in purified water up to a concentration of 30 mg/mL [31]. To the solution thus obtained, 3% v/v chloroform is added, thus proceeding to a first homogenisation for 5 minutes, by means of ULTRA-TURRAX^{®} T-25 (IKA, Germany). The first emulsion is then homogenised under high pressure, using an Emulsiflex EF-B15 (Avestin Inc., Ottawa, Canada), applying a pressure of 13790 mbar (20000 psi) and a number of homogenisation cycles equal to 12. The colloidal dispersion thus obtained was subjected, by means of a rotary evaporator, to a vacuum pressure of 533 mbar (400 mm Hg) for 30 minutes at +40 °C to remove the chloroform. The NP-HSAs were then characterised by size (nm), PDI and zeta potential (mV) by means of Malvern Zetasizer Ultra (Malvern Instruments, Worcestershire, UK). For the size determination and PDI determination, the NP-HSAs were diluted in PBS up to a concentration of 0.1 mg/mL NPs and analysed at +25°C, with multi-angle scattering, and using micro cuvettes (UV-Cuvette, Brand^{®}, Wertheim, Germany). For the zeta potential measurements to determine surface load, the NP-HSAs were diluted in ultra-pure H₂O up to a concentration of 0.1 mg/mL NPs and analysed at +25°C by disposable capillary cells DTS1070 (Malvern Instruments, Worcestershire, UK).

The NP-HSAs were analysed by DLS, from which the mean diameter was found to be 340 nm with a PDI of 0.04.

### Example 4.

### Preparation of NP-HSA derivatised with HSA Cysteine 34 with the linker Maleimide-Glycine-L-lysine-CONH₂ and conjugated to recombinant anti-Her2 Fab using MTG.

For the preparation of the NP-HSAs decorated with anti-HER2 Fab, a recombinant Fab prepared as described in [25] bearing a TQGA sequence sensitive to the action of MTG was used. The external SH groups of the NP-HSAs obtained as in Example 1 or 2 were modified with the peptide linker maleimide-Gly-Lys-NH₂, where the notation Lys-CONH₂ indicates a C-terminal amidated lysine. In relation to the notation reported in formula (I), the "Z" unit is the maleimide group, the glycine amino acid represents the "spacer" and the Lys-CONH₂ therefore represents the "X-NH₂" group. An HSA/linker molar ratio of 1:5 was used for the derivatisation reaction. After 16 hours of incubation at rt and under constant stirring, the modified NPs, termed NP-HSA-Cys-Gly-Lys, were washed three times in H₂O so as to remove the excess reagent and resuspended in phosphate buffer. The NP-HSA-Cys-Gly-Lys were reacted with anti-HER2 Fab, prepared as described [25], containing the TQGA tetrapeptide at the C-Terminal so that it could be conjugated by MTG to the lysines introduced into the NPs. For the conjugation reaction, 40 µg/mL of anti-Her2 Fab was reacted with 1 mg/mL of NP-HSA-Cys-Gly-Lys in the presence of 0.25 U/ml of MTG and in pH 7.3 phosphate buffer, volume 1 mL. The Fab conjugation reaction to the NP-HSA-Cys-Gly-Lys was monitored at different times (from 1h to 16h) by RP-HPLC, analysing the supernatants and evaluating the decrease in the concentration of the free Fab in solution. The analysis in RP-HPLC showed that the amount of Fab conjugated after 16 hours was 10 µg/mg NP-HSA-Cys-Gly-Lys. The NP-HSAs thus obtained were then purified with three wash cycles with H₂O by centrifugation so as to remove the excess MTG and Fab. Finally, they were re-dispersed in PBS and stored at +4°C to be characterised by size (nm), PDI and zeta potential (mV) by means of Malvern Zetasizer Ultra (Malvern Instruments, Worcestershire, UK). An Agilent 1100 Series (Agilent Technologies, Santa Clara, CA) instrument with a C4 Vydac analytical column, 4.6 x 250 mm, 5 µm particle size was used for the analyses in RP-HPLC. For the quantification of the anti-Her2 Fab, the following method was used: mobile phase A: H₂O + 0.1% TFA and mobile phase B ACN + 0.1% TFA, gradient from 30% to 45% B in 25 minutes, flow 0.7 mL/min. For the determination of size and PDI, the NP-HSAs thus obtained were diluted in PBS up to a concentration of 0.1 mg/mL NP-HSA and analysed at +25°C, with multi-angle scattering, and using micro cuvettes (UV-Cuvette, Brand^{®}, Wertheim, Germany). For the zeta potential measurements to determine surface load, the nanoparticles were diluted in ultra-pure H₂O up to a concentration of 0.1 mg/ml NP-HSA and analysed at +25°C by DTS1070 capillary cells (Malvern Instruments, Worcestershire, UK). All the size and Zeta Potential determinations were done in triplicate and represented as mean ± standard deviations.

The NP-HSAs and the NP-HSAs conjugated to the Fab were analysed by DLS, from which it was found that the average diameter passes from 130 nm (PDI 0.04) for the unmodified NP-HSAs, to 145 nm (PDI 0.09) for the NP-HSAs conjugated to the Fab.

### Example 5.

### Preparation of NP-HSA derivatised with HSA cysteine 34 with the linker Maleimide-Gly-Lys-NH₂ and conjugated to recombinant anti-Cripto Fab 10D1 using MTG.

For the preparation of the conjugated NP-HSAs, by means of MTG with the anti-Cripto Fab, the SH groups of the NPs obtained in example 1 were modified with the peptide linker Mal-Gly-Lys-NH2. An HSA/linker molar ratio of 1:5 was used for the functionalisation reaction. After 16 hours of incubation at rt and under constant stirring, the modified NP-HSAs were washed three times in H₂O so as to remove the excess reagent, resuspended in phosphate buffer. The NP-HSA-Cys-Gly-Lys were reacted with the anti-Cripto-1 Fab containing the TQGA tetrapeptide at the C-Terminal so that it could be conjugated by MTG to the lysines introduced into the NP-HSAs. The anti-Cripto Fab [24] was prepared as described [25] using the proprietary sequence of the anti-Cripto-1 antibody 10D1. For the conjugation reaction, 40 µg/mL of anti-Cripto-1 Fab was reacted with 1 mg/mL of HSA-NP in the presence of 0.25 U/ml of MTG and in pH 7.3 phosphate buffer in the volume of 1 mL. The Fab conjugation reaction to the NP-HSA-Cys-Gly-Lys was monitored at different times (from 1h to 16h) by RP-HPLC, analysing the supernatants and evaluating the decrease in the concentration of the free Fab in solution. The analysis in RP-HPLC showed that the amount of Fab conjugated after 16 hours was 10 µg/mg NP-HSA. The nanoparticles were then purified with three wash cycles with H₂O by centrifugation so as to remove the excess MTG and Fab. Finally, they were re-dispersed in PBS and stored at +4°C to be characterised by size (nm), polydispersity (PDI) and zeta potential (mV) by means of Malvern Zetasizer Ultra (Malvern Instruments, Worcestershire, UK). An Agilent 1100 Series (Agilent Technologies, Santa Clara, CA) instrument with a C4 Vydac analytical column, 4.6 x 250 mm, 5 µm particle size was used for the analyses in RP-HPLC. For the quantification of the anti-Her2 Fab, the following method was used: mobile phase A H₂O + 0.1% TFA and mobile phase B ACN + 0.1% TFA, gradient from 30% to 45% B in 25 minutes, flow 0.7 mL/min. For the size measurements and for the determination of the PDI, the nanoparticles were diluted in PBS up to a concentration of 0.1 mg/mL NPs and analysed at +25°C, with multi-angle scattering, and using micro cuvettes (UV-Cuvette, Brand^{®}, Wertheim, Germany). For the zeta potential measurements to determine surface load, the nanoparticles were diluted in ultra-pure H₂O up to a concentration of 0.1 mg/ml NP-HSA and analysed at +25°C by DTS1070 capillary cells (Malvern Instruments, Worcestershire, UK). All the Size and Zeta Potential measurements were done in triplicate and represented as mean ± standard deviations.

The non-derivatised NP-HSAs and the NP-HSAs conjugated to the Fab were analysed by DLS, from which it was found that the average diameter passes from 130 nm (PDI 0.04) for the unmodified NPs, to 145 nm (PDI 0.09) for the NP-HSAs conjugated to the Fab.

### Example 6.

### Preparation of NP-HSA derivatised with HSA cysteine 34 with the linker Br-CH₂-COO2Oc-K-NH₂ and conjugated to the recombinant anti-Her2 Fab using MTG.

For the preparation of the NP-HSAs conjugated, by means of MTG, with the anti-HER2 Fab, the SH groups of the NP-HSAs obtained in example 1 were modified with the peptide linker Br-CH₂-CO-O2Oc-K-NH₂. In relation to the notation reported in formula (I), the "Z" unit is the bromine acetate group, the annotation O2Oc refers to the non-natural amino acid 8-amino-3,6- dioxo-octanoic acid and represents the "spacer", and Lys-NH₂ therefore represents the "X-NH₂" group.

An HSA/linker molar ratio of 1:5 was used for the functionalisation reaction. After 16 hours of incubation at rt and under constant stirring, the modified NP-HSAs, defined as NP-HSA-Cys-O2Oc-Lys, were washed three times in H₂O so as to remove the excess reagent and resuspended in phosphate buffer. The NPs-Cys-O2Oc-Lys were reacted with the anti-HER2 Fab containing the TQGA tetrapeptide at the C-Terminal [32-35] so that it could be conjugated by MTG to the lysines introduced into the NP-HSAs. For the conjugation reaction, 40 µg of anti-Her2 Fab was reacted with 1 mg of HSA NP-HSA-Cys-O2Oc-Lys in the presence of 0.25 U of MTG in a final volume of 1 mL in pH 7.3 phosphate buffer. The Fab conjugation reaction to NPs-Cys-O2Oc-Lys was monitored at different times (from 1h to 16h) by RP-HPLC, analysing the supernatants and evaluating the decrease in the concentration of the free Fab in solution. The analysis in RP-HPLC showed that the amount of Fab conjugated after 16 hours was 10 µg/mg NP-HSA. The nanoparticles were then purified with three wash cycles with H₂O by centrifugation so as to remove the excess MTG and Fab. Finally, they were re-dispersed in PBS and stored at +4°C to be characterised by size (nm), polydispersity (PDI) and zeta potential (mV) by means of Malvern Zetasizer Ultra (Malvern Instruments, Worcestershire, UK). An Agilent 1100 Series (Agilent Technologies, Santa Clara, CA) instrument with a C4 Vydac analytical column, 4.6 x 250 mm, 5 µm particle size was used for the analyses in RP-HPLC. For the quantification of the anti-Her2 Fab, the following method was used: mobile phase A H₂O + 0.1% TFA and mobile phase B ACN + 0.1% TFA, gradient from 30% to 45% B in 25 minutes, flow 0.7 mL/min. For the size measurements and for the determination of the PDI, the NP-HSAs were diluted in PBS up to a concentration of 0.1 mg/mL NPs and analysed at +25°C, with multi-angle scattering, and using micro cuvettes (UV-Cuvette, Brand^{®}, Wertheim, Germany). For the zeta potential measurements to determine surface load, the nanoparticles were diluted in ultra-pure H₂O up to a concentration of 0.1 mg/ml NPs and analysed at +25°C by DTS1070 capillary cells (Malvern Instruments, Worcestershire, UK). All the Size and Zeta Potential measurements were done in triplicate and represented as mean ± standard deviations.

The non-derivatised NP-HSAs and the NP-HSAs conjugated to the Fab were analysed by DLS, from which it was found that the average diameter passes from 130 nm (PDI 0.04) for the unmodified NPs, to 145 nm (PDI 0.09) for the NP-HSAs conjugated to the Fab.

### Example 7.

### Preparation of NP-HSA derivatised with HSA cysteine 34 with the linker Br-CH₂-COO2Oc-K-NH₂ and conjugated to recombinant anti-Cripto-1 Fab 10D1 and anti Her2 Fab using MTG.

For the preparation of the conjugated HSA nanoparticles, by means of MTG, with the two anti-HER2 and anti-Cripto-1 Fabs, the SH groups of the NP-HSAs obtained in example 1 were first modified with the peptide linker Br-CH₂-CO-O2Oc-K-NH₂ as described in the previous example. An HSA/linker molar ratio of 1:5 was used for the functionalisation reaction. After 16 hours of incubation at rt and under constant stirring, the modified NP-HSAs (NP-HSA-Cys-O2Oc-Lys), were washed three times in H₂O so as to remove the excess reagent and resuspended in phosphate buffer. The NP-HSA-Cys-O2Oc-Lys were reacted simultaneously with a 1:1 mixture of the two different Fabs: anti-HER2 and anti-Cripto-1, both containing the TQGA tag at the C-Terminal so that they could be conjugated by MTG to the lysines introduced into the NP-HSAs. For the conjugation reaction, 20 µg of anti-HER2 Fab and 20 ug of anti-Cripto Fab were reacted with 1 mg of HSA NPs-O2Oc-Lys in the presence of 0.25 U of MTG in a final volume of 5 ml in pH 7.3 phosphate buffer. The Fab conjugation reaction to NP-HSA-Cys-O2Oc-Lys was monitored at different times (from 1h to 16h) by RP-HPLC, analysing the supernatants and evaluating the decrease in concentration of the two Fabs in solution. From the analyses in RP-HPLC, it was found that the amount of the two Fab conjugates after 16 hours was 5 µg for the anti Cripto-1 Fab and 2 µg for the trastuzumab Fab for 1 mg NPs. The nanoparticles were then purified with three wash cycles with H₂O by centrifugation so as to remove the unreacted MTG and Fabs. Finally, they were re-dispersed in PBS and stored at +4°C to be characterised by size (nm), polydispersity (PDI) and zeta potential (mV) by means of Malvern Zetasizer Ultra (Malvern Instruments, Worcestershire, UK). An Agilent 1100 Series (Agilent Technologies, Santa Clara, CA) instrument with a C4 Vydac analytical column, 4.6 x 250 mm, 5 µm particle size was used for the analyses in RP-HPLC. For the quantification of the two Fabs, the following method was used: mobile phase A H₂O + 0.1% TFA and mobile phase B ACN + 0.1% TFA, gradient from 30% to 45% B in 25 minutes, flow 0.7 mL/min.

For the size measurements and for the determination of the PDI, the nanoparticles were diluted in PBS up to a concentration of 0.1 mg/mL NPs and analysed at +25°C, with multi-angle scattering, and using micro cuvettes (UV-Cuvette, Brand^{®}, Wertheim, Germany). For the zeta potential measurements to determine surface load, the nanoparticles were diluted in ultra-pure H₂O up to a concentration of 0.1 mg/ml NPs and analysed at +25°C by DTS1070 capillary cells (Malvern Instruments, Worcestershire, UK). All the Size and Zeta Potential measurements were done in triplicate and represented as mean ± standard deviations.

The non-derivatised NP-HSAs and the NP-HSAs conjugated to the Fab were analysed by DLS, from which it was found that the average diameter passes from 130 nm (PDI 0.04) for the unmodified NPs, to 145 nm (PDI 0.09) for the NP-HSAs conjugated to the Fab.

### Example 8

### Chemical synthesis of the Linker Br-CH₂-CO-O2Oc-K-NH₂

The structure of the linker is Br-CH₂-CO-O₂Oc -L-Lys-CONH₂, where the notation Br-CH₂-CO- refers to a residue of bromoacetic acid at the N-terminal ("Z" unit), the notation O2Oc refers to the non-natural amino acid 8-amino-3,6- dioxo-octanoic acid and the notation L-Lys-CONH₂ refers to the natural C-terminal amidated L-lysine amino acid. The peptide was prepared by solid phase synthesis using an MBHA Rink Amide resin (IRIS BIOTECH, 0.74 mmol/g) using the Fmoc methodology. The synthesis was performed on a scale of 590 µmoles. The resin was washed in dimethylformamide (DMF, Romil, DELTEK) 3 times (5 mL x 3) and treated for 10 minutes with 20% v/v piperidine (ROMIL, DELTEK) in DMF to remove the Fmoc group from the resin. Next, the L-lysine amino acid was attached in the form of an Fmoc-L-Lys (Boc)-OH derivative (IRIS BIOTECH) dissolved at the concentration of 0.5 M in DMF. The amino acid, used at an excess of 5 times, was pre-activated with HATU/DIEA, excesses 1:2 mol/mol (IRIS BIOTECH) as described in the literature [36] and left in contact with the resin for 1 hour at RT. The resin was washed with DMF 3 times and then treated with 5 mL of 40% v/v piperidine in DMF for 20 minutes. The resin was drained and washed with DMF 3 times. The resin was then treated with the derivative Fmoc- O₂Oc-OH (8-(9-Fluorenylmethyloxycarbonyl- amino)-3,6- dioxaoctanoic acid, IRIS BIOTECH) dissolved at the concentration of 0.5 M in DMF and pre-activated with excess HBTU/DIEA 1:2 mol/mol as described in the literature [36] for 1 hour at RT. The resin was finally washed 3 times with DMF and treated with 5 mL of 40% v/v piperidine in DMF for 20 minutes. The resin was drained and washed with DMF 3 times. The resin was finally treated with 25 equivalents of Bromoacetic acid at the concentration of 1 M in DCM (Dichloromethane, ROMIL, DELTEK) with added DIEA (1:1 mol/mol) for 2 hours at RT. The resin was finally washed with DCM, DMF and again DCM and then dried under vacuum. For the peptide detachment, the resin was treated with 5 mL of TFA(trifluoroacetic acid)/Tis(Tri-isopropyl-silane)/H₂O 90/5/5 (v/v/v) solution for 3 hours at RT. The resin was filtered off, the peptide precipitated with cold ethyl ether, isolated by centrifugation and lyophilised by H₂O and ACN (Acetonitrile, ROMIL, DELTEK). The crude material was purified by preparative RP-HPLC and finally characterised by LC-MS using an Xbridge C18 column (50 x 2.1 mm ID, 5µm) on an LC-MS ESI-TOF system (6230 ESI-TOF mass spectrometer coupled to HPLC 1290 Infinity system. Gradient from 1% solvent B (ACN, 0.05% TFA) to 80% B in 10 minutes at a flow of 0.2 mL/min. Solvent A was H₂O, 0.05% TFA. Approximately 180 mg of pure product (95%, HPLC), 74% yield, was obtained. The determined experimental mass was 410.13 amu, in excellent agreement with the experimental mass of 410.12 amu.

### Example 9

### Synthesis of the linker Mal-Gly-Lys-CONH₂

The structure of the linker is Mal-Gly-Lys-CONH₂, where the notation Mal refers to the 6-Maleimidohexanoic group at the N-terminal ("Z" unit), the notation Gly refers to the natural glycine amino acid and the notation L-Lys-CONH₂ refers to the natural C-terminal amidated L-lysine amino acid. The peptide was prepared by solid phase synthesis using an MBHA Rink Amide resin (0.74 mmol/g) using the Fmoc methodology. The synthesis was performed on a scale of 590 µmoles. The resin was washed in DMF 3 times (5 mL x 3) and treated for 10 minutes with 20% v/v piperidine in DMF to remove the Fmoc group from the resin. Next, the L-lysine amino acid was attached in the form of an Fmoc-L-Lys (Boc)-OH derivative dissolved at the concentration of 0.5 M in DMF. The amino acid, used at an excess of 5 times, was pre-activated with HATU/DIEA, excesses 1:2 mol/mol (IRIS BIOTECH) as described in the literature [36] and left in contact with the resin for 1 hour at RT. The resin was washed with DMF 3 times and then treated with 5 mL of 40% v/v piperidine in DMF for 20 minutes. The resin was drained and washed with DMF 3 times. The resin was then treated with the derivative Fmoc-Gly-OH (9-Fluorenylmethyloxycarbonyl-glycine, IRIS BIOTECH) dissolved at the concentration of 0.5 M in DMF and pre-activated with 1:2 mol/mol excess HBTU/DIEA as described in the literature [36] for 1 hour at RT. The resin was finally washed 3 times with DMF and treated with 5 mL of 40% v/v piperidine in DMF for 20 minutes. The resin was drained and washed with DMF 3 times. The resin was finally treated with 25 equivalents of 6-Maleimidohexanoic acid (Sigma-Aldrich code: 755842) at 1 M concentration in DMF with DIEA added (1:1 mol/mol) for 2 hours at RT. The resin was finally washed with DCM, DMF and again DCM and then dried under vacuum. For the peptide detachment, the resin was treated with 5 mL of TFA(trifluoroacetic acid)/Tis(Tri-isopropyl-silane)/H₂O 90/5/5 (v/v/v) solution for 3 hours at RT. The resin was filtered off, the peptide precipitated with cold ethyl ether, isolated by centrifugation and lyophilised by H₂O and ACN. The crude material was purified by preparative RP-HPLC and finally characterised by LC-MS using an Xbridge C18 column (50 x 2.1 mm ID, 5µm) on an LC-MS ESI-TOF system (6230 ESI-TOF mass spectrometer coupled to the HPLC 1290 Infinity system. Gradient from 1% solvent B (ACN, 0.05% TFA) to 80% B in 10 minutes at a flow of 0.2 mL/min. Solvent A was H₂O, 0.05% TFA.

Approximately 163 mg of pure product (95%, HPLC), about 70% yield, was obtained. The determined experimental mass was 394.64 amu, in excellent agreement with the experimental mass of 394.61 amu.

### Example 10

### Competitive binding of the recombinant Her2 bond between NP-HSAs decorated with trastuzumab Fab and trastuzumab.

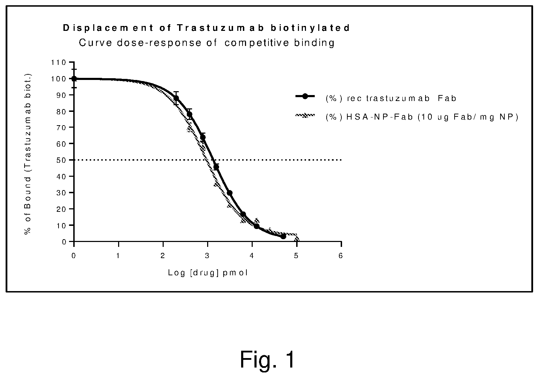

The quantity of antagonist which binds a receptor can be detected indirectly through a displacement test using the same ligand or a surrogate thereof marked with a reporter. The displacement test was performed using NP-HSAs functionalised with trastuzumab Fab, prepared as described in **Example 4 and Example 6,** the recombinant receptor Her2-Fc (Recombinant human ErbB2 Fc Chimera/R&D 1129-ER-050) immobilised on the surface of multiwell plates, and the antibody biotinylated trastuzumab. The experiment was conducted by measuring the ability of the NP-HSAs functionalised with trastuzumab Fab to displace the binding of the biotinylated trastuzumab to the receptor immobilised on the surface of 96-well multiwell plates. The experiment was carried out using, in parallel experiments, Fab not bound to the NP-HSAs. The Her2-Fc receptor was immobilised on 96-well polystyrene multiwell plates by incubation of solutions (100 µL/well) at 0.5 µg/mL concentration in phosphate buffer overnight at 4°C. The blocking was carried out by incubation with a 1% solution of BSA (Bovine Serum Albumin/ Merck A7906, 300 µL/mL) in phosphate buffer (2 hours incubation at 37 °C). Biotinylated trastuzumab solutions (provided by the CNR Institute of Biostructures and Bioimaging) were then added to the various wells at a fixed concentration of 100 pM containing NP-HSA-Fab (10 µg/mg of NP-HSA) at increasing molar concentrations of Fab comprised between 0.39 nM (about 2 µg/mL NP-HSA-Fab) and 100 nM (0.5 mg/mL NP-HSA-Fab). The experiment was performed in quadruplicate (4 wells per single concentration). The detection of biotinylated trastuzumab bound to the immobilised receptor was carried out using a solution of Streptavidin-Peroxidase (Merck S5512) at a concentration of 50 ng/mL (100 µL/well) and subsequent incubation with the substrate TMB (3,3',5,5'-tetramethylbenzidine/ Merck T0440). The reaction was blocked with H₂SO₄ 1N and the absorbance was read at 450 nm using a multiplate reader (model 680, BIO-RAD). The absorbance values obtained at 450 nm were subtracted from the blank values (absorbance of the wells not immobilised with Her2-Fc), averaged and expressed as a percentage compared to the control of the biotinylated trastuzumab alone. The data were fitted with a sigmoid type curve using GraphPad Prism 6.0 software to determine the inhibition IC₅₀. The experiments were repeated 3 times and averaged. The IC₅₀ data are reported in **Table 2** below. The inhibition curve is shown in **Figure 1****.** The data demonstrate that the HP-NSAs functionalised with Fab have the same ability to bind to the isolated Fab receptor.

**Table 2. HER2 binding competition IC50 values obtained with trastuzumab Fab and for the same Fab bound to human albumin particles.**

| | **Recombinant Trastuzumab Fab** | **NP-HSA-Fab (10 µg/mg HSA)** |
|---|---|---|
| **Mean IC₅₀ (nM)** ± **SD** | 2.38±0.17 | 2.39±0.27 |

### Example 11

### Binding to Her2-positive BT474 cells of NP-HSA conjugated to trastuzumab Fab and unconjugated recombinant Fab.

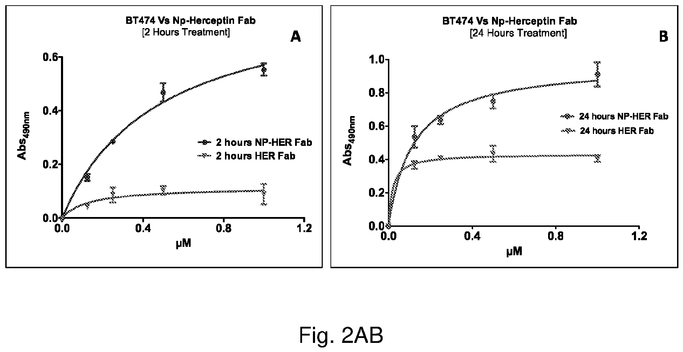

Her2-positive BT474 cells, a human breast cancer cell line, were plated at a cell density of 10,000 cells/well in a 96-well plate (Falcon^{®} 96-well Clear TC-Treated Microplates, Thermo Scientific). After incubating overnight at +37°C with 5% CO₂, the cells were fixed with 4% formaldehyde for 15 minutes at room temperature, then washed with PBS 1X twice and finally treated with 3% hydrogen peroxide for 10 minutes at room temperature. After washing with PBS 1X twice, the cells were treated with PBS containing 3% BSA, PBS-A (Sigma Aldrich, A3294) for 1 hour at room temperature. In duplicate, the wells were treated with the NP-HSAs conjugated with trastuzumab Fab, prepared as described in **Example 4 and Example 6,** with the NP-HSAs and with the trastuzumab Fab, in a concentration range comprised between 0.12 µM and 1.0 µM and brought to a final volume of 100 µL/well with PBS-A. The cells were incubated with the samples for 2 hours and for 24 hours at room temperature and after 3 washes in PBS 1X, were treated with an HRP-conjugated mouse IgG (Sigma-Aldrich) capable of recognising the human Fab. Finally, the wells were treated with GAM-HRP (Sigma-Aldrich) at a dilution of 1:1000 for 1 hour at room temperature. The cells were washed three times with PBS and treated with OPD (Sigma Aldrich P9187) in the dark for 5 min (100 µL/well). The reaction was blocked using H₂S0₄ 2.5 M (50 µL/well). The absorbance of the samples was read at 490 nm using a BioTek microplate reader (Winooski, VT, USA). The values were averaged, blank subtracted (values in wells treated with non-functionalised NP-HSAs) and plotted using GraphPad prism 6.0 to determine the binding constant. The binding curves are shown in **Figure 2AB****.** The data demonstrate that, with the same concentration of Fab, the NP-HSAs functionalised with trastuzumab Fab are able to bind the receptor on the cells with greater efficiency than the Fab isolated both after 2 hours and after 24 hours.

### Example 12

### Binding to Her2-positive BT474 cells and Her2-negative MDA-MB-231 cells of NP-HSA conjugated to trastuzumab Fab and unconjugated recombinant Fab.

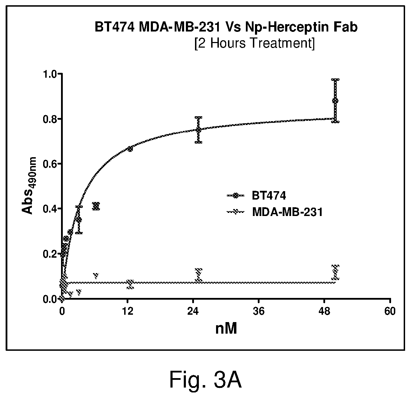

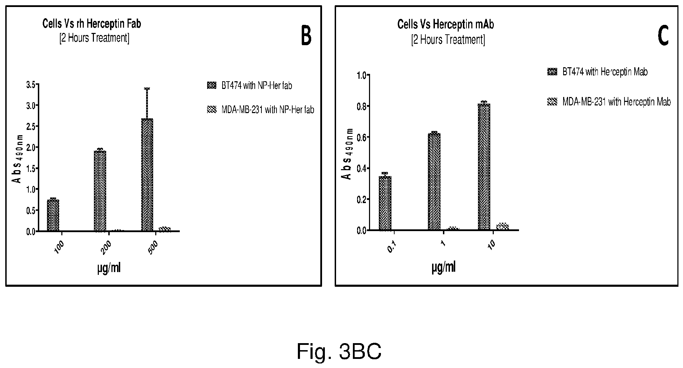

Her2-positive BT474 cells were plated at a cell density of 10,000 cells/well in a 96-well plate (Falcon^{®} 96-well Clear TC-Treated Microplates, Thermo Scientific). Her2-negative MDA-MB-231 cells, a human breast cancer cell line, were plated at a cell density of 5,000 cells/well in an identical second plate. After incubating overnight at +37°C with 5% CO2, the cells were fixed with 4% formaldehyde for 15 minutes at room temperature, then washed with PBS 1X twice and finally treated with 3% hydrogen peroxide for 10 minutes at room temperature. After washing with PBS 1X twice, the cells were treated with PBS containing 3% BSA, PBS-A (Sigma Aldrich, A3294) for 1 hour at room temperature. In duplicate, the wells were treated with the trastuzumab Fab-conjugated NP-HSAs, prepared as described in **Example 4 and Example 6,** in a concentration comprised between 0.03 µM and 50 nM. To verify the expression of the receptor on the BT474 cells and the lack of expression thereof on the MDA-MB-231 cells, experiments were conducted in parallel with non-functionalised NP-HSAs at the same concentrations, with the trastuzumab Fab at the concentration of 500, 200 and 100 µg/mL and the whole trastuzumab antibody at the concentration of 10, 1.0 and 0.1 µg/mL, dissolved in a final volume of 100 µL/well of PBS-A. The cells were incubated with the samples for 2 hours and for 24 hours at room temperature and after 3 washes in PBS 1X, they were treated with a mouse IgG conjugated with HRP (Sigma-Aldrich) capable of recognising the human Fab. Finally, the wells were treated with GAM-HRP (Sigma-Aldrich) at a dilution of 1: 1000 for 1 hour at room temperature. The cells were washed three times with PBS and treated with OPD (Sigma Aldrich P9187) in the dark for 5 min (100 µL/well). The reaction was blocked using H₂S0₄ 2.5 M (50 µL/well). The absorbance of the samples was read at 490 nm using a BioTek microplate reader (Winooski, VT, USA). The values were averaged, blank subtracted (values in wells treated with non-functionalised NP-HSAs) and plotted using GraphPad prism 6.0 to determine the binding constant. The binding curves are shown in **Figure 3ABC****.** The data in **Figure 3A** demonstrate that the NP-HSAs functionalised with the trastuzumab Fab are capable of binding to the BT474 cells expressing the Her2 receptor on their surface while negligibly binding non-receptor-expressing MDA-MB-231 line cells. The data in **Figure 3B and 3C** obtained with the recombinant Fab at very high concentrations and with the whole antibody demonstrate the lack of Her2 receptor expression on the MDA-MB-231 control cells. The non-functionalised NP-HSAs did not give detectable signals.

### Example 13.

### Binding to NTERA cells of NP-HSA conjugated to recombinant anti-Cripto Fab 10D1 and unconjugated recombinant Fab.

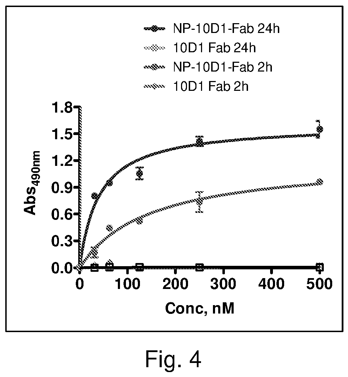

Cripto-1-positive cells, NTERA, were plated at a cell density of 5000 cells per well in a 96-well plate (Falcon^{®} 96-well Clear TC-Treated Microplates, Thermo Scientific). After being incubated overnight at +37°C with 5% CO2, the cells were fixed with 4% formaldehyde for 15 minutes at room temperature, then washed with PBS 1X twice and finally treated with 3% hydrogen peroxide for 10 minutes at room temperature. After washing with PBS 1X twice, the cells were treated with 3% PBS-A for 1 hour at room temperature. The duplicate wells were treated with the NP-HSAs functionalised with the anti-Cripto Fab 10D1, prepared as described in **Example 5,** and the isolated Fab in a concentration range comprised between 0.000117 µM and 0.5 µM and with the empty NP-HSAs and brought to a final volume of 100 µL/well with a 3% PBS-BSA solution. The cells were incubated with the samples for 2 hours and 24 hours at room temperature and after 3 washes in PBS 1X, they were treated with an anti-human mouse IgG conjugated with HRP (Sigma-Aldrich) capable of recognising the Fab. Finally, GAM-HRP (Sigma-Aldrich) was added to each well at a dilution of 1: 1000 for 1 hour at room temperature. The cells were washed three times with PBS and then treated with OPD in the dark for 5 min (100 µL/ well). Subsequently, the reaction was blocked using 2.5 M H₂S0₄. The absorbance of the samples was then read at 490 nm using a BioTek microplate reader (Winooski, VT, USA). The binding curves are shown in **Figure 4** and demonstrate that the NP-HSAs functionalised with the Fab 10D1 are able to bind to the NTERA cells in a dose-response and saturable manner both after 2 hours and after 24 hours while the isolated Fab is not able to bind under the same conditions. The non-functionalised NP-HSAs did not give detectable signals.

### Example 14.

### Binding to Her2-positive BT474 cells and Her2-negative MDA-MB-231 cells of bispecific NP-HSA functionalised with recombinant anti-Cripto Fab 10D1 and recombinant trastuzumab Fab.

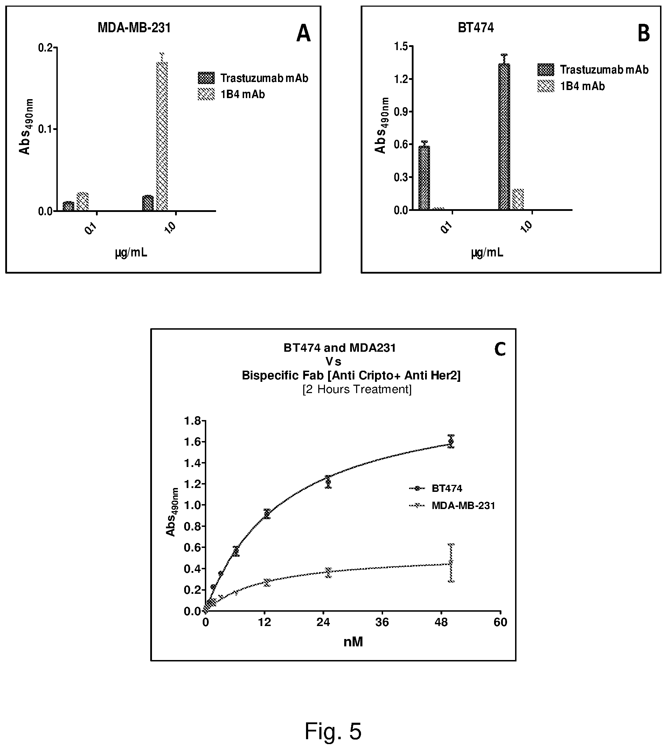

Cripto-1 and Her2-positive cells, BT474, were plated at a cell density of 10,000 cells per well in a 96-well plate (Falcon^{®} 96-well Clear TC-Treated Microplates, Thermo Scientific). Cripto-1-positive and Her2-negative MDA-MB-231 cells were instead plated at a cell density of 5,000 cells per well in a 96-well plate. After being incubated overnight at +37°C with 5% CO2, the cells were fixed with 4% formaldehyde for 15 minutes at room temperature, then washed with PBS 1X twice and finally treated with 3% hydrogen peroxide for 10 minutes at room temperature. After washing with PBS 1X twice, the cells were treated with 3% PBS-A for 1 hour at room temperature. Duplicate wells were treated with the NP-HSAs functionalised with the two recombinant anti-Cripto-1 Fab 10D1 and trastuzumab Fab, prepared as described in **Example 7,** and having Fab 10D1 density 5.0 µg/mg NP-HSA and trastuzumab Fab density 2.0 µg/mg NP-HSA. The functionalised NP-HSAs were used at increasing concentrations, expressed as equivalent concentrations of total Fab, between 0.097 nM (0.343 µg/mL NP-HSA) and 50 nM (175 µg/mL NP-HSA). In parallel the same cells were treated with whole 1B4 antibodies (Anti-Cripto-1, [30] and trastuzumab at 0.1 and 1.0 µg/mL concentration. All the samples were used in a final volume of 100 µL/well with a 3% PBS-BSA solution. The cells were incubated with the samples for 2 hours at room temperature and after 3 washes in PBS 1X, they were treated with an anti-human mouse IgG conjugated with HRP (Sigma-Aldrich) capable of recognising the Fab. Finally, GAM-HRP (Sigma-Aldrich) was added in each well at a dilution of 1: 1000 for 1 hour at room temperature. The cells were washed three times with PBS and then treated with OPD in the dark for 5 min (100 µL/ well). Subsequently, the reaction was blocked using 2.5 M H₂S0₄. The absorbance of the samples was then read at 490 nm using a BioTek microplate reader (Winooski, VT, USA). **Figure 5AB** shows the binding data of the whole B4 antibodies anti-Cripto-1 and trastuzumab to the two cell lines at concentrations of 0.1 and 1.0 µg/mL and demonstrate that the BT474 cells express the Her2 receptor and to a lesser extent Cripto-1 and that the MDA-MB-231 cells express Cripto-1 only to a small extent. **Figure 5C** shows the dose-response binding curves and demonstrate that the NP-HSAs functionalised with the two recombinant Fabs are able to bind both Her2-negative BT474 and MDA-MB-231 cells thanks to the presence of the anti-Cripto-1 Fab alone.

The non-functionalised NP-HSAs did not give detectable signals.

### Example 15.

### Binding to Her2-positive BT474 cells and Her2-negative MDA-MB-231 cells of bispecific NP-HSA functionalised with recombinant anti-Cripto Fab 10D1 and recombinant trastuzumab Fab compared to the bond obtained with a mixture of the two NP-HSAs.

Cripto-1 and Her2-positive cells, BT474, were plated at a cell density of 10,000 cells per well in a 96-well plate (Falcon^{®} 96-well Clear TC-Treated Microplates, Thermo Scientific). Cripto-1-positive and Her2-negative MDA-MB-231 cells were instead plated at a cell density of 5,000 cells per well in a 96-well plate. After being incubated overnight at +37°C with 5% CO2, the cells were fixed with 4% formaldehyde for 15 minutes at room temperature, then washed with PBS 1X twice and finally treated with 3% hydrogen peroxide for 10 minutes at room temperature. After washing with PBS 1X twice, the cells were treated with 3% PBS-A for 1 hour at room temperature. The duplicate wells were treated at increasing concentrations of total Fab between 24 pM and 12 nM with the NP-HSA-functionalised bispecific NP-HSAs prepared as described in **Example 7** at a density of 3.0 µg/mg NP-HSA (10D1) and 2 µg/mg NP-HSA (trastuzumab), with the NP-HSAs functionalised with Fab 10D1 alone as described in **Example 5** at a density of 10.0 µg/mg NP-HSA at increasing concentrations of Fab between 24 pM and 12 nM, with the NP-HSAs functionalised with trastuzumab Fab alone at a density of 10.0 µg/mg NP-HSA prepared as described in **Example 4** and in **Example 6** at increasing concentrations of Fab between 24 pM and 12 nM, the two anti-Cripto-1 mAb trastuzumab and 1B4 [30] used both at the concentration of 1.0 µg/mL and 0.1 µg/mL. All the samples were used in a final volume of 100 µL/well with a 3% PBS-BSA solution. The cells were incubated with the samples for 2 hours at room temperature and after 3 washes in PBS 1X, they were treated with an anti-human mouse IgG conjugated with HRP (Sigma-Aldrich) capable of recognising the Fab. Finally, GAM-HRP (Sigma-Aldrich) was added to each well at a dilution of 1: 1000 for 1 hour at room temperature. The cells were washed three times with PBS and then treated with OPD in the dark for 5 min (100 µL/ well). Subsequently, the reaction was blocked using 2.5 M H₂S0₄. The absorbance of the samples was then read at 490 nm using a BioTek microplate reader (Winooski, VT, USA). The dose-response binding curves are shown in **Figure 6ABCD** and demonstrate that the bispecific NP-HSAs functionalised with the two recombinant Fabs are able to bind with the same efficiency as the combination of the two NP-HSAs functionalised with the two separate Fabs to both the BT474 and MDA-MB-231 cells. The non-functionalised NP-HSAs did not give detectable signals.

### Example 16.

### Preparation of NP-HSAs functionalised with fluorescein and with the antibody anti-Cripto-1 Fab 10D1 and NTERA2 cell binding experiment expressing Cripto-1 by cytofluorimetry.

The NP-HSAs were prepared as described in **Example 1.** 5.0 mg of NP-HSA were suspended in 1.0 mL of 0.1 M borate buffer, pH 9.0. 0.14 mg of fluorescein isothiocyanate (FITC, Sigma Aldrich code F4274) were added to the suspension, solubilised in 50 µL of anhydrous DMSO, so as to reach an HSA:FITC ratio of 1:5 mol/mol. The suspension was kept stirring at 37°C for 3 hours.

The NP-HSA conjugation reaction to FITC was monitored at different times (1h to 3h) by RP-HPLC, analysing the supernatant and evaluating the decrease in the concentration of the free FITC in solution. The analysis in RP-HPLC showed that the amount of FITC conjugated after 3 hours of reaction was 8 µg/mg NPs. The nanoparticles were then purified with three wash cycles with H₂O by centrifugation so as to remove the excess FITC. Finally, they were re-dispersed in PBS and stored at +4 °C. The nanoparticles were defined as FITC-NP-HSA. An aliquot of the FITC-NP-HSA (4.0 mg) was used for conjugation by MTG with the anti-Cripto-1 Fab 10D1 to obtain the FITC-NP-HSA conjugated to the two Fabs (FITC-NP-HSA-Fab). 4.0 mg of FITC-NP-HSA were treated with the peptide linker Br-CH₂-CO-O2Oc-K-NH₂ (see **Example 6 and Example 8)** using an HSA/linker ratio of 1:5 mol/mol. After 16 hours of incubation at rt and under constant stirring, the modified NPs (FITC-NP-HSA-Lys) were washed three times in H₂O so as to remove the excess reagent and then resuspended in phosphate buffer. The FITC-NP-HSA-Lys were functionalised with the anti-Cripto Fab 10D1 (see **Example 7);** the functionalisation was performed using MTG exploiting the presence of the TQGA tag at the C-Terminal. For the functionalisation reactions, 40 µg of Fab was used in the presence of 0.25 U of MTG, in a final volume of 1 mL of phosphate buffer pH 7.3. The Fab conjugation reaction to the FITC-NP-HSA-Lys was monitored at different times (from 1h to 16h) by RP-HPLC, analysing the supernatants and evaluating the decrease in the concentration of the free Fab in solution. The analysis in RP-HPLC showed that the amount of Fab conjugated after 16 hours was 10 µg/mg NP-HSA. The NP-HSAs thus obtained, referred to as FITC-NP-HSA-Fab, were then purified with three wash cycles with H₂O by centrifugation so as to remove the excess MTG and Fab. Finally, they were re-dispersed in PBS and stored at +4°C to be characterised by size (nm), polydispersity (PDI) and zeta potential (mV) by means of Malvern Zetasizer Ultra (Malvern Instruments, Worcestershire, UK). An Agilent 1100 Series (Agilent Technologies, Santa Clara, CA) instrument with a C4 Vydac analytical column, 4.6 x 250 mm, 5 µm particle size was used for the analyses in RP-HPLC. For the quantification of the Fab in solution, the following method was used: mobile phase A H₂O + 0.1% TFA and mobile phase B ACN + 0.1% TFA, gradient from 30% to 45% B in 25 minutes, flow 0.7 ml/min.

For the cytofluorimetric analysis, the NTERA2 cells were plated at a density of 40% in 10%FBS DMEM/F12 with the addition of glutamine and antibiotics. The next day, the cells were washed with PBS and serum-free medium was added containing the following nanoparticles FITC-NP-HSA, FITC-NP-HSA-Fab. The NP-HSAs marked with Fab without FITC were used as a negative control. The amount of NP used was normalised with respect to the FITC-NP-HSA-Fab, used so as to have an anti-Cripto Fab concentration of 1000 ng/mL, 100 ng/mL and 10 ng/mL. The cells were incubated with the NPs for 4 hours at 37°C, then washed and resuspended in 0.5% BSA PBS. The samples were acquired at the "BD FACS ARIAIII cell sorter", and analysed for size and intensity of FITC. The results report the percentage of FITC-positive cells (gate P3) and the average fluorescence intensity of the total population analysed (P1 MFI). At 100 ng/mL, a slight signal was observed in favour of the FITC-NP-HSA-Fab (1.5%) compared to the FITC-NP-HSA without Fab (0.5%). At 1000 ng/mL the signal is much more intense for both preparations. In terms of number of marked cells, it is observed that there is no substantial difference between the NPs decorated with Fab (53.5%) and those not decorated (63.6%). The fluorescence intensity observed with the FITC-NP-HSA-Fab is much higher compared to that observed with the FITC-NP-HSA with an MFI of 3313 and 752, respectively. A 4.4-times higher fluorescence signal is then observed in the NP-HSA-treated cells decorated with Fab compared to the undecorated NP-HSAs, suggesting that the presence of the anti-Cripto-1 Fab allows for greater NP-HSA input into some cells.

### References

[1]Sleep, D.; Cameron, J.; Evans, L.R., Albumin as a versatile platform for drug half-life extension. Biochim Biophys Acta, 2013, 1830, (12), 5526-5534.
[2]Hoogenboezem, E.N.; Duvall, C.L., Harnessing albumin as a carrier for cancer therapies. Adv Drug Deliv Rev, 2018, 130, 73-89.
[3]Birn, H.; Christensen, E.I., Renal albumin absorption in physiology and pathology. Kidney Int, 2006, 69, (3), 440-449.
[4]Birn, H.; Fyfe, J.C.; Jacobsen, C.; Mounier, F.; Verroust, P.J.; Orskov, H.; Willnow, T.E.; Moestrup, S.K.; Christensen, E.I., Cubilin is an albumin binding protein important for renal tubular albumin reabsorption. J Clin Invest, 2000, 105, (10), 1353-1361.
[5]Merlot, A.M.; Kalinowski, D.S.; Richardson, D.R., Unraveling the mysteries of serum albumin-more than just a serum protein. Front Physiol, 2014, 5, 299.
[6]Miller, M.A.; Gadde, S.; Pfirschke, C.; Engblom, C.; Sprachman, M.M.; Kohler, R.H.; Yang, K.S.; Laughney, A.M.; Wojtkiewicz, G.; Kamaly, N.; Bhonagiri, S.; Pittet, M.J.; Farokhzad, O.C.; Weissleder, R., Predicting therapeutic nanomedicine efficacy using a companion magnetic resonance imaging nanoparticle. Sci Transl Med, 2015, 7, (314), 314ra183.
[7]Commisso, C.; Davidson, S.M.; Soydaner-Azeloglu, R.G.; Parker, S.J.; Kamphorst, J.J.; Hackett, S.; Grabocka, E.; Nofal, M.; Drebin, J.A.; Thompson, C.B.; Rabinowitz, J.D.; Metallo, C.M.; Vander Heiden, M.G.; Bar-Sagi, D., Macropinocytosis of protein is an amino acid supply route in Ras-transformed cells. Nature, 2013, 497, (7451), 633-637.
[8]Campbell, P.M.; Der, C.J., Oncogenic Ras and its role in tumor cell invasion and metastasis. Semin Cancer Biol, 2004, 14, (2), 105-114.
[9]Kamphorst, J.J.; Nofal, M.; Commisso, C.; Hackett, S.R.; Lu, W.; Grabocka, E.; Vander Heiden, M.G.; Miller, G.; Drebin, J.A.; Bar-Sagi, D.; Thompson, C.B.; Rabinowitz, J.D., Human pancreatic cancer tumors are nutrient poor and tumor cells actively scavenge extracellular protein. Cancer Res, 2015, 75, (3), 544-553.
[10]Hauser, C.A.; Stockler, M.R.; Tattersall, M.H., Prognostic factors in patients with recently diagnosed incurable cancer: a systematic review. Support Care Cancer, 2006, 14, (10), 999-1011.
[11]Chatterjee, M.; Ben-Josef, E.; Robb, R.; Vedaie, M.; Seum, S.; Thirumoorthy, K.; Palanichamy, K.; Harbrecht, M.; Chakravarti, A.; Williams, T.M., Caveolae-Mediated Endocytosis Is Critical for Albumin Cellular Uptake and Response to Albumin-Bound Chemotherapy. Cancer Res, 2017, 77, (21), 5925-5937.
[12]Suzuoki, M.; Miyamoto, M.; Kato, K.; Hiraoka, K.; Oshikiri, T.; Nakakubo, Y.; Fukunaga, A.; Shichinohe, T.; Shinohara, T.; Itoh, T.; Kondo, S.; Katoh, H., Impact of caveolin-1 expression on prognosis of pancreatic ductal adenocarcinoma. Br J Cancer, 2002, 87, (10), 1140-1144.
[13]Li, R.; Yang, H.; Jia, D.; Nie, Q.; Cai, H.; Fan, Q.; Wan, L.; Li, L.; Lu, X., Fusion to an albumin-binding domain with a high affinity for albumin extends the circulatory half-life and enhances the in vivo antitumor effects of human TRAIL. J Control Release, 2016, 228, 96-106.
[14]Zhang, Y.; Sun, T.; Jiang, C., Biomacromolecules as carriers in drug delivery and tissue engineering. Acta Pharm Sin B, 2018, 8, (1), 34-50.
[15]Kuhlmann, M.; Hamming, J.B.R.; Voldum, A.; Tsakiridou, G.; Larsen, M.T.; Schmokel, J.S.; Sohn, E.; Bienk, K.; Schaffert, D.; Sorensen, E.S.; Wengel, J.; Dupont, D.M.; Howard, K.A., An Albumin-Oligonucleotide Assembly for Potential Combinatorial Drug Delivery and Half-Life Extension Applications. Mol Ther Nucleic Acids, 2017, 9, 284-293.
[16] Elzoghby, A.O.; Samy, W.M.; Elgindy, N.A., Albumin-based nanoparticles as potential controlled release drug delivery systems. J Control Release, 2012, 157, (2), 168-182.
[17]Wagner, S.; Rothweiler, F.; Anhorn, M.G.; Sauer, D.; Riemann, I.; Weiss, E.C.; Katsen-Globa, A.; Michaelis, M.; Cinatl, J., Jr.; Schwartz, D.; Kreuter, J.; von Briesen, H.; Langer, K., Enhanced drug targeting by attachment of an anti alphav integrin antibody to doxorubicin loaded human serum albumin nanoparticles. Biomaterials, 2010, 31, (8), 2388-2398.
[18]Greally, M.; Kelly, C.M.; Cercek, A., HER2: An emerging target in colorectal cancer. Curr Probl Cancer, 2018, 42, (6), 560-571.
[19]da Silva, J.L.; Cardoso Nunes, N.C.; Izetti, P.; de Mesquita, G.G.; de Melo, A.C., Triple negative breast cancer: A thorough review of biomarkers. Crit Rev Oncol Hematol, 2020, 145, 102855.
[20]Sandomenico, A.; Ruvo, M., Targeting Nodal and Cripto-1: Perspectives Inside Dual Potential Theranostic Cancer Biomarkers. Curr Med Chem, 2019, 26, (11), 1994-2050.
[21]Sharma, A.; Kaur, A.; Jain, U.K.; Chandra, R.; Madan, J., Stealth recombinant human serum albumin nanoparticles conjugating 5-fluorouracil augmented drug delivery and cytotoxicity in human colon cancer, HT-29 cells. Colloids Surf B Biointerfaces, 2017, 155, 200-208.
[22]Zong, Y.; Wu, J.; Shen, K., Nanoparticle albumin-bound paclitaxel as neoadjuvant chemotherapy of breast cancer: a systematic review and meta-analysis. Oncotarget, 2017, 8, (10), 17360-17372.
[23]Zhang, J.Y.; He, B.; Qu, W.; Cui, Z.; Wang, Y.B.; Zhang, H.; Wang, J.C.; Zhang, Q., Preparation of the albumin nanoparticle system loaded with both paclitaxel and sorafenib and its evaluation in vitro and in vivo. J Microencapsul, 2011, 28, (6), 528-536.
[24]Foca, G.; laccarino, E.; Foca, A.; Sanguigno, L.; Untiveros, G.; Cuevas-Nunez, M.; Strizzi, L.; Leonardi, A.; Ruvo, M.; Sandomenico, A., Development of conformational antibodies targeting Cripto-1 with neutralizing effects in vitro. Biochimie, 2019, 158, 246-256.
[25]Selis, F.; Sandomenico, A.; Cantile, M.; Sanna, R.; Calvanese, L.; Falcigno, L.; Dell'Omo, P.; Esperti, A.; De Falco, S.; Foca, A.; Caporale, A.; laccarino, E.; Truppo, E.; Scaramuzza, S.; Tonon, G.; Ruvo, M., Generation and testing of engineered multimeric Fabs of trastuzumab. Int J Biol Macromol, 2020.
[26]Behdani, M.; Zeinali, S.; Khanahmad, H.; Karimipour, M.; Asadzadeh, N.; Azadmanesh, K.; Khabiri, A.; Schoonooghe, S.; Habibi Anbouhi, M.; Hassanzadeh-Ghassabeh, G.; Muyldermans, S., Generation and characterization of a functional Nanobody against the vascular endothelial growth factor receptor-2; angiogenesis cell receptor. Mol Immunol, 2012, 50, (1-2), 35-41.
[27]Pruszynski, M.; Koumarianou, E.; Vaidyanathan, G.; Revets, H.; Devoogdt, N.; Lahoutte, T.; Zalutsky, M.R., Targeting breast carcinoma with radioiodinated anti-HER2 Nanobody. Nucl Med Biol, 2013, 40, (1), 52-59.
[28]Oliveira, S.; Schiffelers, R.M.; van der Veeken, J.; van der Meel, R.; Vongpromek, R.; van Bergen En Henegouwen, P.M.; Storm, G.; Roovers, R.C., Downregulation of EGFR by a novel multivalent nanobody-liposome platform. J Control Release, 2010, 145, (2), 165-175.
[29] Weber, C.; Coester, C.; Kreuter, J.; Langer, K., Desolvation process and surface characterisation of protein nanoparticles. Int J Pharm, 2000, 194, (1), 91-102.
[30]Langer, K.; Balthasar, S.; Vogel, V.; Dinauer, N.; von Briesen, H.; Schubert, D., Optimization of the preparation process for human serum albumin (HSA) nanoparticles. Int J Pharm, 2003, 257, (1-2), 169-180.
[31]Lomis, N.; Westfall, S.; Farahdel, L.; Malhotra, M.; Shum-Tim, D.; Prakash, S., Human Serum Albumin Nanoparticles for Use in Cancer Drug Delivery: Process Optimization and In Vitro Characterization. Nanomaterials (Basel), 2016, 6, (6).
[32]Caporale, A.; Monti, A.; Selis, F.; Sandomenico, A.; Tonon, G.; Ruvo, M.; Doti, N., A comparative analysis of catalytic activity and stability of microbial transglutaminase in controlled denaturing conditions. J Biotechnol, 2019, 302, 48-57.
[33]Caporale, A.; Selis, F.; Sandomenico, A.; Jotti, G.S.; Tonon, G.; Ruvo, M., The LQSP tetrapeptide is a new highly efficient substrate of microbial transglutaminase for the site-specific derivatization of peptides and proteins. Biotechnol J, 2015, 10, (1), 154-161.
[34]Doti, N.; Caporale, A.; Monti, A.; Sandomenico, A.; Selis, F.; Ruvo, M., A recent update on the use of microbial transglutaminase for the generation of biotherapeutics. World J Microbiol Biotechnol, 2020, 36, (4), 53.
[35]Selis, F.; Foca, G.; Sandomenico, A.; Marra, C.; Di Mauro, C.; Saccani Jotti, G.; Scaramuzza, S.; Politano, A.; Sanna, R.; Ruvo, M.; Tonon, G., Pegylated Trastuzumab Fragments Acquire an Increased in Vivo Stability but Show a Largely Reduced Affinity for the Target Antigen. Int J Mol Sci, 2016, 17, (4), 491.
[36] Caporale, A.; Doti, N.; Sandomenico, A.; Ruvo, M., Evaluation of combined use of Oxyma and HATU in aggregating peptide sequences. J Pept Sci, 2017, 23, (4), 272-281.

## Claims

1. Serum albumin nanoparticles (Alb-NP) decorated with at least one decoration chain comprising:
• a linker, bound to the nanoparticles by means of an -S- thioether bond, and
• at least one biological molecule,
wherein the at least one biological molecule is bound to the linker through an amide bond formed by means of a transamidation (or transglutamination) reaction, mediated by the enzyme transglutaminase,
said nanoparticles having the following formula (I): 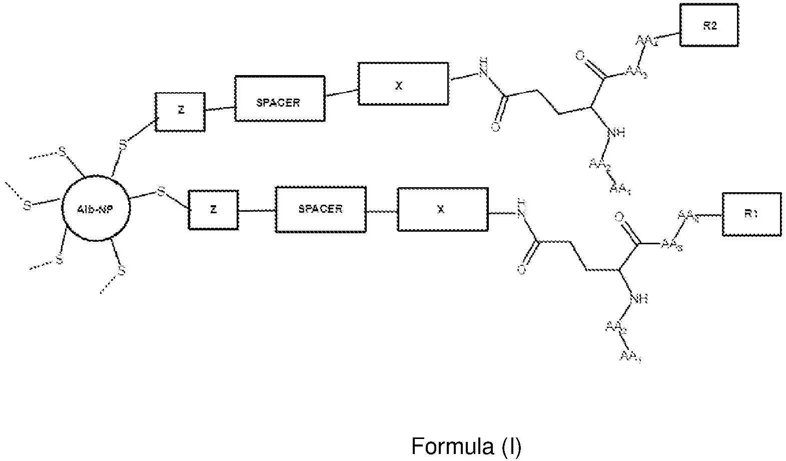 wherein:
Alb-NP is a serum albumin nanoparticle containing, on the surface, thiol functionalities (-SH) reacted with an electrophilic group of Z to form the -S- thioether bond;
Z-spacer-X-NH- is a linker wherein:
Z derives from a functional group containing an electrophilic group capable of reacting with the -SH group of the albumin, thereby forming the -S- thioether bond, wherein said functional group is selected from: 2-bromoacetic acid, 3-bromopropanoic acid, 3-chloropropanoic acid, 4-bromobutyric acid, 5-chlorobutyric acid, 5-bromopentanoic acid, 5-chloropentanoic acid, 4-bromomethyl benzoic acid, 4-chloromethyl benzoic acid, 2-maleimidoacetic acid, 3-maleimidopropionic acid, 4-maleimidobutyric acid, 5-maleimidopentanoic acid, 6-maleimidohexanoic acid, 3-maleimidobenzoic acid, 4-maleimidobenzoic acid, 4-(2-N-Maleimido)methyl benzoic acid, 1-bromoacetic acid and 1-chloroacetic acid;
the spacer is selected from:
• -NH-(CH₂-O)ₙ-CH₂-CO-, with n from 2 to 10, preferably 2, 3, 4 and 5;
• -NH-(CH₂-CH₂-O)ₙ-CH₂-CO-, with n from 2 to 10, preferably 2, 3, 4 and 5;
or the spacer is a Yₘ group,
wherein Y is selected from:
• an -NH-(CH₂)ₙ-CO- amino acid, with n from 3 to 10, preferably between 3 and 5, more preferably with n = 3, 4 or 5;
• glycine, alanine;
• and combinations thereof;
m is a number between 1 and 5;
wherein the -NH- group of the spacer forms an amide bond with the carboxylic group of the precursor of Z and the -CO- group of the spacer forms an amide bond with the -NH- group of the subsequent unit X;
the -X-NH- group is selected from:
• -NH-(CH₂)ₙ-NH- with n ranging between 3 and 10, preferably 3, 4 and 5;
• -NH-(O-CH₂)ₙ-NH- with n ranging between 2 and 10, preferably 2, 3, 4 and 5;
• -NH-(O-CH₂-CH₂)ₙ-NH- with n ranging between 2 and 10, preferably 2, 3, 4 and 5;
• L-lysine amino acid;
• L-ornithine amino acid;
• C-terminal amidated L-lysine amino acid;
• C-terminal amidated L-ornithine amino acid;
wherein the two -NH- groups of the -X-NH- group form an amide bond, respectively, with the -CO- group of the spacer and with the glutamine of a consensus sequence with the formula (VI):
AA₁-AA₂-Q-AA₃-AA₄ Formula (VI)
wherein:
AA₁ is leucine (L; Leu) or is absent;
AA₂ is leucine (L; Leu) or is threonine (T; Thr);
Q is glutamine with the formula -CO-(CH₂)₂-CH-(NH)-CO-;
AA₃ is serine (S; Ser) or is glycine (G; Gly);
AA₄ is proline (P; Pro) or alanine (A; Ala) or is absent;
preferably, AA₁ and AA₄ are not simultaneously absent;
R1 and R2, different from or identical to each other, are selected from: a Fab, an scFv, a nanobody (NB), an antibody and combinations thereof,
or
said albumin nanoparticles having the formula (II): 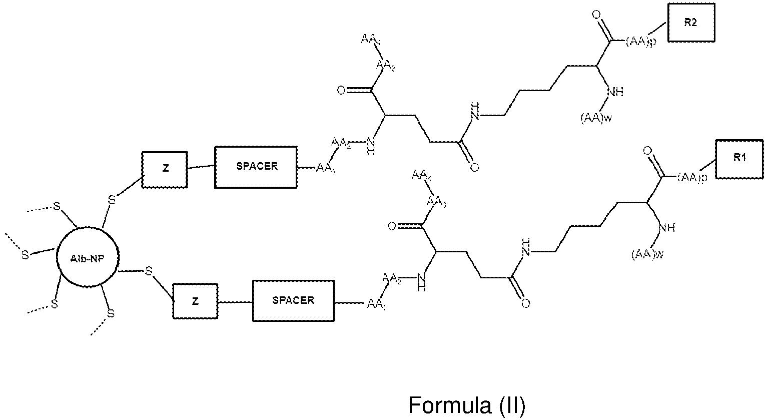 wherein,
Z-Spacer- AA₁-AA₂-Q-AA₃-AA₄ is a linker;
Alb-NP, Z, spacer, R1 and R2 are as defined for the formula (I);
AA₁-AA₂-Q-AA₃-AA₄, with Q equal to glutamine with the formula -CO-(CH₂)₂-CH-(NH)-CO-, is as defined for the formula (I) and is bound to the spacer by means of an amide bond between the -CO- terminal of the spacer and an -NH- group of the amino acid AA₁, if present, or AA₂;
the peptide sequence containing a lysine (K; Lys) has the formula (IX):
(AA)_{w}-K-(AA)ₚ Formula (IX)
wherein
w and p are whole numbers comprised between 0 and 8, preferably between 1 and 5, with the condition that w and p are never simultaneously equal to 0;
AA indicates an amino acid selected from: alanine (A; Ala), tyrosine (Y; Tyr), phenylalanine (F; Phe), glycine (G; Gly), tryptophan (W; Trp) and serine (S; Ser);
K is a lysine (Lys).

2. The albumin nanoparticles according to claim 1, wherein the nanoparticles are human albumin nanoparticles (NP-HSA) or bovine albumin nanoparticles (NP-BSA).

3. The albumin nanoparticles according to claim 1 or 2, wherein the nanoparticles have an average diameter (or Z-average size) of 100-500 nm, preferably 100-400 nm or 300-400 nm, measured with the Dynamic Light Scattering (DLS) technique.

4. The albumin nanoparticles according to any one of claims 1 to 3, loaded with at least one cytotoxic drug selected from: 5-FU, capecitabin, cytarabine, fludarabine, cladribine, paclitaxel, doxorubicin, daunorubicin, epirubicin, docetaxel, vinblastine, vincristine, vinorelbine, mercaptopurine, methotrexate, raltitrexed, etoposide, teniposide, camptothecin, irinotecan, topotecan and combinations thereof.

5. The albumin nanoparticles according to any one of claims 1 to 4, wherein R1 and R2 are different from each other and are two different types of Fab: Fab1 and Fab2, two different types of scFv: scFv1 and scFv2, two different types of NBs: NB1 and NB2, two different types of antibody: Ab1 and Ab2, or R1 and R2 are mixed combinations of biological molecules, preferably Fab1 and scFv1, Fab1 and NB1, scFv1 and NB1, or Ab1 and NB1.

6. The albumin nanoparticles according to any one of claims 1 to 5, wherein the antibody is a monoclonal antibody selected from: antibody DI17E6, Trastuzumab, Pertuzumab, Cetuximab, anti-Cripto-1 monoclonal antibody, preferably anti-Cripto-1 antibody 1B4 or anti-Cripto-1 antibody 10D1; the Fab is selected from: recombinant Fab of Trastuzumab, of Pertuzumab, of an anti-Cripto-1 monoclonal antibody, preferably selected from anti-Cripto-1 antibody 11B4 and anti-Cripto-1 antibody 10D1; the scFv is a functional fragment of an antibody selected from: antibody DI17E6, Trastuzumab, Pertuzumab, Cetuximab, an anti-Cripto-1 monoclonal antibody, preferably anti-Cripto-1 antibody 1B4 or anti-Cripto-1 antibody 10D1; the nanobodies (NBs) are selected from an anti-VEGFR2 NB, preferably NB 3VGR19; anti-Her2 NB, preferably NB 5F7GGC; anti-EGFR NB, preferably NB EGa1.

7. The albumin nanoparticles according to any one of claims 1 to 6, wherein Z derives from a functional group selected from: 1-bromoacetic acid, 1-chloroacetic acid and 6-maleimidohexanoic acid.

8. The albumin nanoparticles according to any one of claims 1 to 7, wherein the spacer is - NH-(CH₂-CH₂-O)ₙ-CH₂-CO-, with n comprised between 2 and 5, or it is glycine.

9. The albumin nanoparticles according to any one of claims 1 to 8, wherein the X-NH- group is selected from: L-lysine amino acid and C-terminal amidated L-lysine amino acid.

10. The albumin nanoparticles according to any one of claims 1 to 9, wherein the consensus sequence is selected from: LQSP, TQGA, LLQG.

11. The albumin nanoparticles according to any one of claims 1 to 8, wherein in the formula (IX)
(AA)_{w}-K-(AA)ₚ
w is equal to 0 and p is equal to 1-3; preferably, w is equal to 0 and p is equal to 3, more preferably it is KAYA, KGYA, KSYA, KAFA, KGFA, KSFA, KAWA, KGWA, KSWA, KAYG, KGYG, KSYG, KAFG, KGFG, KSFG, KAWG, KGWG, KSWG, KAYS, KGYS, KSYS, KAFS, KGFS, KSFS, KAWS, KGWS, KSWS.

12. The serum albumin nanoparticles according to any one of claims 1 to 11, wherein the nanoparticles are covalently bound on the surface to a fluorescent dye, preferably fluorescein isothiocyanate.

13. The serum albumin nanoparticles according to any one of claims 1 to 12, selected from:

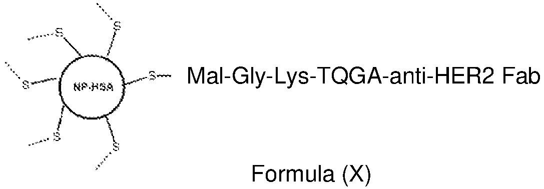

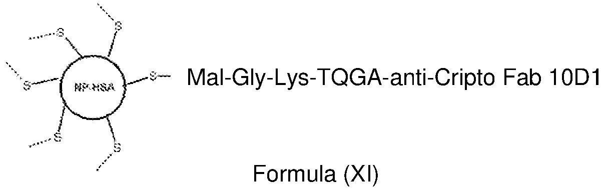

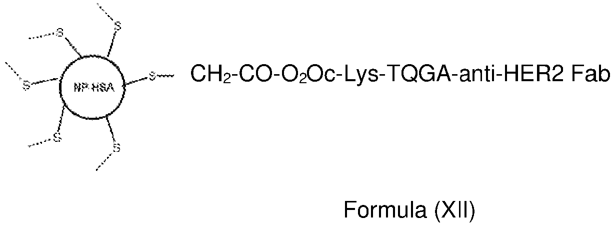

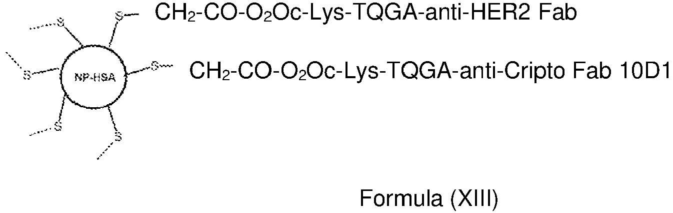

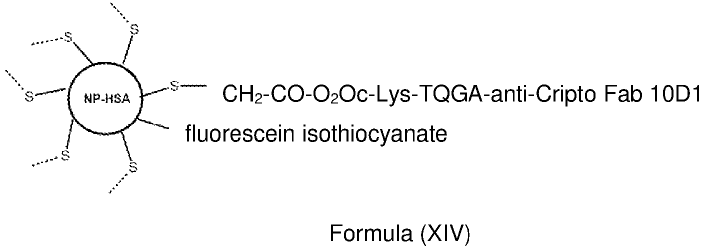

14. A process for preparing the albumin nanoparticles according to any one of claims 1 to 13, wherein the at least one biological molecule is bound to the linker through an amide bond formed by means of a transamidation (or transglutamination) reaction, mediated by the enzyme transglutaminase, between:
(i) the NH₂ residue of an X group of a linker and the -CO-NH₂ residue of a glutamine comprised in a peptide consensus sequence inserted in the at least one biological molecule; or
(ii) the -NH₂ residue of a lysine comprised in a peptide sequence inserted in the at least one biological molecule and the -CO-NH₂ residue of a glutamine inserted in a consensus sequence which is part of the linker.

## Patentansprüche

1. Serum-Albuminnanopartikel (Alb-NP), die mit mindestens einer Dekorationskette dekoriert sind, umfassend:
· einen Linker, gebunden mit den Nanopartikeln mittels einer -S- Thioetherbindung und
· mindestens ein biologisches Molekül,
wobei das mindestens eine biologische Molekül mit dem Linker durch eine Amidbindung gebunden ist, die durch eine durch die Enzymtransglutaminase gemittelte Transamidierungs- (oder Transglutaminierungs-)Reaktion gebildet ist,
wobei die Nanopartikel die folgende Formel (I) aufweisen: 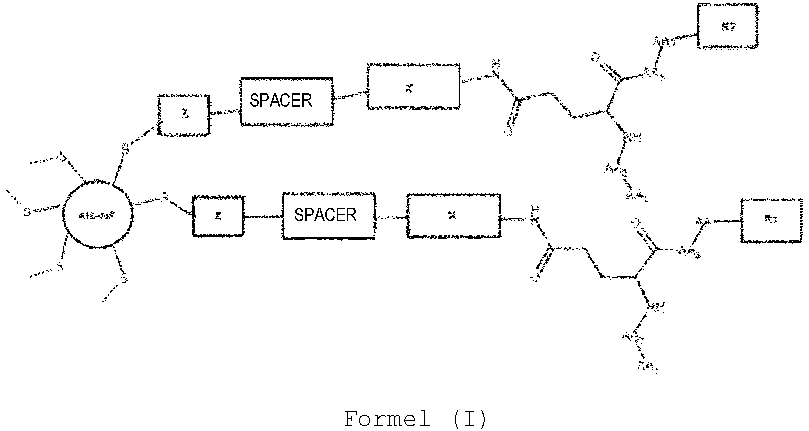 wobei:
Alb-NP ein Serum-Albuminnanopartikel ist, der auf der Oberfläche Thiolfunktionen (-SH) enthält, die mit einer elektrophilen Gruppe Z reagierten, um die -S-Thioetherbindung zu bilden;
Z-Spacer-X-NH- ein Linker ist, wobei:
Z von einer funktionalen Gruppe abgeleitet ist, die eine elektrophile Gruppe enthält, die in der Lage ist, mit der -SH-Gruppe des Albumins zu reagieren, wodurch die - S-Thioetherbindung gebildet wird, wobei diese funktionale Gruppe ausgewählt ist aus: 2-Bromessigsäure, 3-Brompropansäure, 3-Chlorpropansäure, 4-Brombuttersäure, 5-Chlorbuttersäure, 5-Brompentansäure, 5-Chlorpentansäure, 4-Brommethyl-Benzosäure, 4-Chlormethyl-Benzosäure, 2-Maleimido-Essigsäure, 3-Maleimidopropionsäure, 4-Maleimidobuttersäure, 5-Maleimidopentansäure, 6-Maleimidohexansäure, 3-Maleimidobenzosäure, 4-Maleimidobenzosäure, 4-(2-N-Maleimodo)methyl-Benzosäure, 1-Bromessigsäure und 1-Chloressigsäure;
wobei der Spacer ausgewählt ist aus:
• -NH-(CH₂-O)ₙ-CH₂-CO-, mit n von 2 bis 10, vorzugsweise 2, 3, 4 und 5;
• -NH-(CH₂-CH₂-O)ₙ-CH₂-CO-, mit n von 2 bis 10, vorzugsweise 2, 3, 4 und 5; oder der Spacer ist eine Gruppe Yₘ,
wobei Y ausgewählt ist aus:
• einer -NH-(CH₂)ₙ-CO-Aminosäure mit n von 3 bis 10, vorzugsweise zwischen 3 und 5, bevorzugter mit n = 3, 4 oder 5;
• Glycin, Alanin;
• und einer Kombination von diesen;
m eine Zahl zwischen 1 und 5 ist;
wobei die -NH-Gruppe des Spacers eine Amidbindung mit der Carboxylgruppe des Präkursors von Z bildet und die -CO-Gruppe des Spacers eine Amidbindung mit der -NH-Gruppe der nachfolgenden Einheit X bildet;
wobei die -X-NH-Gruppe ausgewählt ist aus:
• -NH-(CH₂)ₙ-NH- mit n im Bereich zwischen 3 und 10, vorzugsweise 3, 4 und 5;
• -NH-(O-CH₂)ₙ-NH- mit n im Bereich zwischen 2 und 10, vorzugsweise 2, 3, 4 und 5;
• -NH-(O-CH₂-CH₂)ₙ-NH- mit n im Bereich zwischen 2 und 10, vorzugsweise 2, 3, 4 und 5;
• L-Lysin-Aminosäure;
• L-Ornithin-Aminosäure;
• C-terminal amidierter L-Lysin-Aminosäure;
• C-terminal amidierter L-Ornithin-Aminosäure;
wobei die beiden -NH-Gruppen der -X-NH-Gruppe eine Amidbindung jeweils mit der -CO-Gruppe des Spacers und dem Glutamin einer Konsensussequenz mit der folgenden Formel (VI) bilden:
**AA₁-AA₂-Q-AA₃-AA₄** Formel (VI)
wobei:
AA₁ Leucin (L; Leu) ist oder fehlt;
AA₂ Leucin (L; Leu) ist oder Threonin (T; Thr) ist;
Q Glutamin mit der Formel -CO-(CH₂)₂-CH-(NH)-CO- ist;
AA₃ Serin (S; Ser) oder Glycin (G; Gly) ist;
AA₄ Prolin (P; Pro) oder Alanin (A; Ala) ist oder fehlt; vorzugsweise fehlen AA₁ und AA₄ nicht gleichzeitig;
R1 und R2, die sich voneinander unterscheiden oder identisch sind, sind ausgewählt aus: einem Fab, einem scFv, einem Nanobody (NB), einem Antikörper oder einer Kombination dieser
oder
wobei die Albuminnanopartikeln die folgende Formel (II) aufweisen: 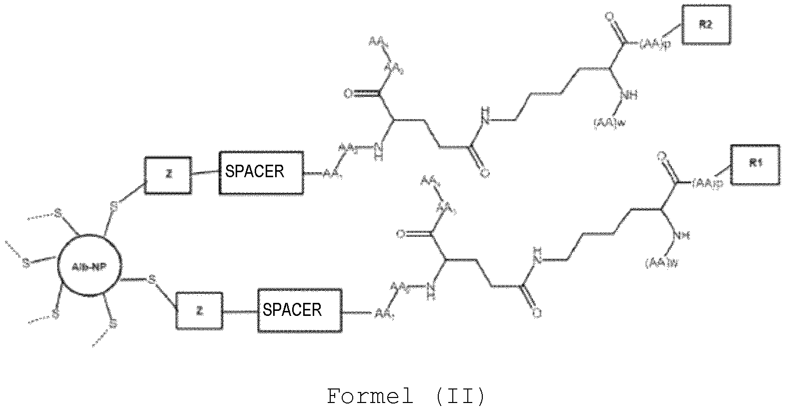 wobei
Z-Spacer- AA₁-AA₂-Q-AA₃-AA₄ ein Linker ist;
Alb-NP, Z, Spacer, R1 und R2 gemäß der Definition für die Formel (I) sind;
AA₁-AA₂-Q-AA₃-AA₄ mit Q gleich Glutamin mit der Formel CO-(CH₂)₂-CH-(NH)-CO- ist gemäß der Definition für die Formel (I) und ist mit dem Spacer mittels einer Amidbindung zwischen dem -CO-Terminus des Spacers und einer -NH-Gruppe der Aminosäure AA₁, wenn vorhanden, oder AA₂ gebunden;
die ein Lysin (K; Lys) enthaltende Peptidsequenz weist die folgende Formel (IX) auf:
**(AA)_{w}-K-(A.A)ₚ** Formel (IX)
wobei
w und p ganze Zahlen zwischen 0 und 8, vorzugsweise zwischen 1 und 5 sind, mit der Bedingung, dass w und p niemals gleichzeitig gleich 0 sind;
AA eine Aminosäure angibt, ausgewählt aus: Alanin (A; Ala), Tyrosin (Y; Tyr), Phenylanalin (F; Phe), Glycin (G; Gly), Tryptophan (W; Trp) und Serin (S; Ser);
K Lysin (Lys) ist.

2. Albuminnanopartikel nach Anspruch 1, wobei die Nanopartikel Human-Albuminnanopartikel (NP-HSA) oder Rinder-Albuminnanopartikel (NP-BSA) sind.

3. Albuminnanopartikel nach Anspruch 1 oder 2, wobei die Nanopartikel einen mittleren Durchmesser (oder Z-Durchschnittsgröße) von 100-500 nm, vorzugsweise 100-400 nm oder 300-400 nm, gemessen mit der Technik der dynamischen Lichtstreuung (DLS), aufweisen.

4. Albuminnanopartikel nach einem der Ansprüche 1 bis 3, die mit mindestens einem zytotoxischen Arzneimittel beladen sind, ausgewählt aus: 5-FU, Capecitabin, Cytarabin, Fludarabin, Cladribin, Paclitaxel, Doxorubicin, Daunorubicin, Epirubicin, Docetaxel, Vinblastin, Vincristin, Vinorelbin, Mercaptopurin, Methotrexat, Raltitrexed, Etoposid, Teniposid, Camptothecin, Irinotecan, Topotecan und Kombinationen von diesen.

5. Albuminnanopartikel nach einem der Ansprüche 1 bis 4, wobei R1 und R2 voneinander unterschiedlich und zwei unterschiedliche Arten von Fab: Fabl und Fab2, zwei unterschiedliche Arten von scFv: scFv1 und scFv2, zwei unterschiedliche Arten von NBs: NB1 und NB2, zwei unterschiedliche Arten von Antikörpern: Ab1 und Ab2 sind, oder R1 und R2 gemischte Kombinationen biologischer Moleküle, vorzugsweise Fabl und scFv1, Fabl und NB1, scFv1 und NB1 oder Ab1 und NB1, sind.

6. Albuminnanopartikel nach einem der Ansprüche 1 bis 5, wobei der Antikörper ein monoklonaler Antikörper ist, ausgewählt aus: Antikörper DI17E6, Trastuzumab, Pertuzumab, Cetuximab, monoklonalem Anti-Cripto-1-Antikörper, vorzugsweise Anti-Cripto-1-Antikörper 1B4 oder Anti-Cripto-1-Antikörper 10D1; der Fab ist ausgewählt, aus: rekombinantem Fab von Trastuzumab, von Pertuzumab, eines monoklonalen Anti-Cripto-1-Antikörpers, vorzugsweise ausgewählt aus Anti-Cripto-1-Antikörper 11B4 und Anti-Cripto-1-Antikörper 10D1; der scFv ist ein funktionelles Fragment eines Antikörpers, ausgewählt aus: Antikörper DI17E6, Trastuzumab, Pertuzumab, Cetuximab, einem monoklonalen Anti-Cripto-1-Antikörpers, vorzugsweise Anti-Cripto-1-Antikörper 1B4 oder Anti-Cripto-1-Antikörper 10D1; die Nanobodies (NBs) sind aus einem Anti-VEGFR2 NB, vorzugsweise NB 3VGR19 ausgewählt; anti-Her2 NB, vorzugsweise NB 5F7GGC; anti-EGFR NB, vorzugsweise NB EGal.

7. Albuminnanopartikel nach einem der Ansprüche 1 bis 6, wobei Z von einer funktionalen Gruppe abgeleitet ist, ausgewählt aus: 1-Bromessigsäure, 1-Chloressigsäure und 6-Maleimidohexansäure.

8. Albuminnanopartikel nach einem der Ansprüche 1 bis 7, wobei der Spacer -NH-(CH₂-CH₂-O)ₙ-CH₂-CO- ist, mit n, enthalten zwischen 2 und 5, oder Glycin ist.

9. Albuminnanopartikel nach einem der Ansprüche 1 bis 8, wobei die X-NH-Gruppe ausgewählt ist aus: L-Lysin-Aminosäure und C-terminal amidierter L-Lyson-Aminosäure.

10. Albuminnanopartikel nach einem der Ansprüche 1 bis 9, wobei die Konsensussequenz ausgewählt ist aus: LQSP, TQGA, LLQG.

11. Albuminnanopartikel nach einem der Ansprüche 1 bis 8, wobei in der Formel (IX)
**(AA)_{w}-K-(AA)ₚ**
w gleich 0 und p gleich 1-3 ist, vorzugsweise w gleich 0 und p gleich 3 ist, noch bevorzugter KAYA, KGYA, KSYA, KAFA, KGFA, KSFA, KAWA, KGWA, KSWA, KAYG, KGYG, KSYG, KAFG, KGFG, KSFG, KAWG, KGWG, KSWG, KAYS, KGYS, KSYS, KAFS, KGFS, KSFS, KAWS, KGWS, KSWS ist.

12. Serum-Albuminnanopartikel nach einem der Ansprüche 1 bis 11, wobei die Nanopartikel an der Oberfläche kovalent an einen Fluoreszenzfarbstoff, vorzugsweise Fluoresceinisothiocyanat, gebunden sind.

13. Serum-Albuminnanopartikel nach einem der Ansprüche 1 bis 12, ausgewählt aus:

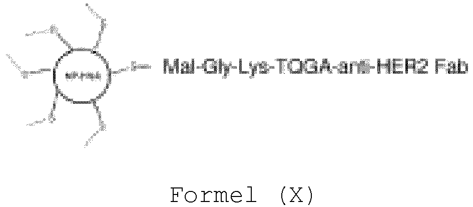

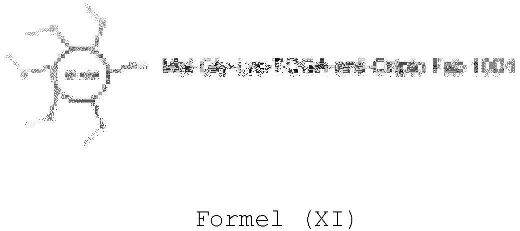

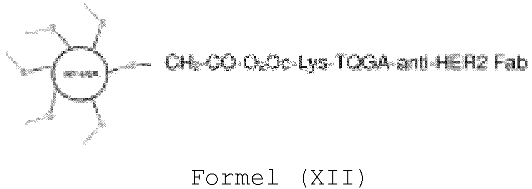

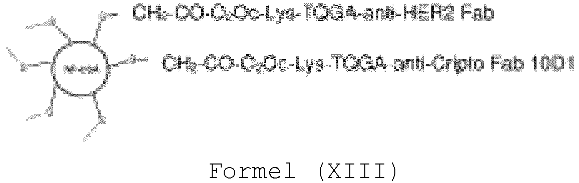

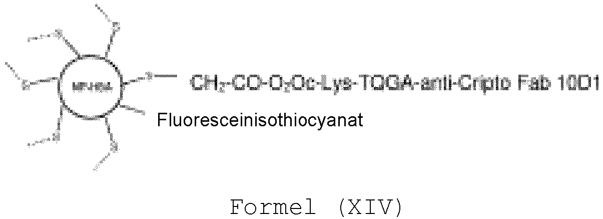

14. Verfahren zur Herstellung der Albuminnanopartikel nach einem der Ansprüche 1 bis 13, wobei das mindestens eine biologische Molekül mit dem Linker durch eine Amidbindung gebunden ist, die durch eine durch die Enzymtransglutaminase gemittelte Transamidierungs-(oder Transglutaminierungs-)Reaktion gebildet ist, zwischen:
(i) dem NH₂-Rest einer X-Gruppe eines Linkers und dem - CO-NH₂-Rest eines Glutamins, der in einer in das mindestens eine biologische Molekül eingefügten Peptidkonsenssequenz enthalten ist;
oder
(ii) dem -NH₂-Rest eines Lysins, das in einer in das mindestens eine biologische Molekül eingefügten Peptidsequenz enthalten ist, und dem -CO-NH₂-Rest eines Glutamins, das in einer Konsensussequenz eingefügt ist, die Teil des Linkers ist.

## Revendications

1. Nanoparticules d'albumine sérique (Alb-NP) décorées d'au moins une chaîne de décoration, comprenant :
· un coupleur, lié aux nanoparticules au moyen d'une liaison thioéther -S-, et
· au moins une molécule biologique,
où l'au moins une molécule biologique est liée au coupleur par une liaison amide formée au moyen d'une réaction de transamidation (ou de transglutamination), médiée par l'enzyme transglutaminase,
lesdites nanoparticules ayant la formule suivante (I) : 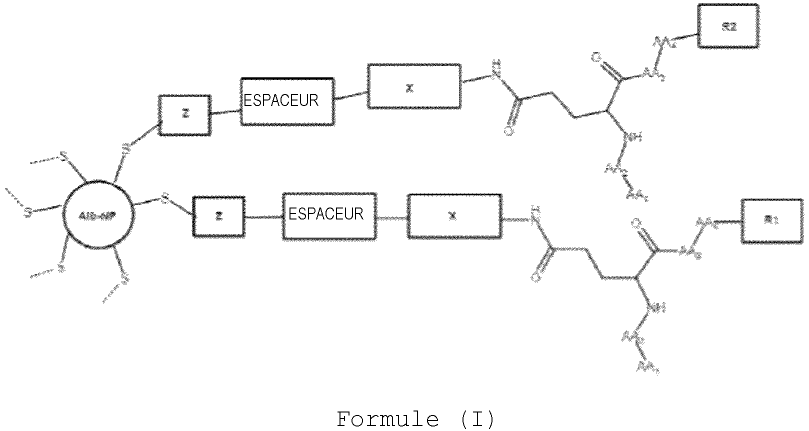 où :
Alb-NP est une nanoparticule d'albumine sérique contenant, sur la surface, des fonctions thioles (-SH) mises à réagir avec un groupe électrophile de Z pour former la liaison thioéther -S- ;
Z-espaceur-X-NH- est un coupleur où :
Z dérive d'un groupe fonctionnel contenant un groupe électrophile capable de réagir avec le groupe -SH de l'albumine, formant ainsi la liaison thioéther -S-, où ledit groupe fonctionnel est choisi parmi : l'acide 2-bromoacétique, l'acide 3-bromopropanoïque, l'acide 3-chloropropanoïque, l'acide 4-bromobutyrique, l'acide 5-chlorobutyrique, l'acide 5-bromopentanoïque, l'acide 5-chloropentanoïque, l'acide 4-bromométhyl benzoïque, l'acide 4-chlorométhyl benzoïque, l'acide 2-maléimidoacétique, l'acide 3-maléimidopropionique, l'acide 4-maléimidobutyrique, l'acide 5-maléimidopentanoïque, l'acide 6-maléimidohexanoïque, l'acide 3-maléimidobenzoïque, l'acide 4-maléimidobenzoïque, l'acide 4-(2-NMaléimido)méthylbenzoïque, l'acide 1-bromoacétique et l'acide 1-chloroacétique ;
l'espaceur est choisi parmi :
• -NH-(CH₂-O)ₙ-CH₂-CO-, avec n compris entre 2 et 10, de préférence 2, 3, 4 et 5 ;
• -NH-(CE₂-CH₂-O)ₙ-CH₂-CO-, avec n compris entre 2 et 10, de préférence 2, 3, 4 et 5 ; ou l'espaceur est un groupe Yₘ,
où Y est choisi parmi :
• un acide aminé NH-(CH₂)ₙ-CO-, avec n compris entre 3 et 10, de préférence entre 3 et 5, plus préférablement avec n = 3, 4 ou 5 ;
• une glycine, une alanine ;
• et leurs combinaisons ;
m est un nombre compris entre 1 et 5 ;
où le groupe -NH- de l'espaceur forme une liaison amide avec le groupe carboxylique du précurseur de Z et le groupe -CO- de l'espaceur forme une liaison amide avec le groupe -NH- de l'unité suivante X ;
le groupe -X-NH- est choisi parmi :
• -NH-(CH₂)ₙ-NH- avec n compris entre 3 et 10, de préférence 3, 4 et 5 ;
• -NH-(O-CH₂)ₙ-NH- avec n compris entre 2 et 10, de préférence 2, 3, 4 et 5 ;
• -NH-(O-CH₂-CH₂)ₙ-NH- avec n compris entre 2 et 10, de préférence 2, 3, 4 et 5 ;
• un acide aminé L-lysine ;
• un acide aminé L-ornithine ;
• un acide aminé L-lysine amidé sur C-terminal ;
• un acide aminé L-ornithine amidé sur C-terminal ;
où les deux groupes -NH- du groupe -X-NH- forment une liaison amide, respectivement, avec le groupe -CO- de l'espaceur et avec la glutamine d'une séquence consensus de formule (VI) :
**AA₁-AA₂-Q-AA₃-AA₄** Formule (VI)
où :
AA₁ est la leucine (L ; Leu) ou est absent ;
AA₂ est la leucine (L ; Leu) ou est la thréonine (T ; Thr) ;
Q est la glutamine de formule -CO-(CH₂)₂-CH-(NH)-CO- ;
AA₃ est la sérine (S ; Ser) ou la glycine (G ; Gly) ;
AA₄ est la proline (P ; Pro) ou l'alanine (A ; Ala) ou est absent ;
de préférence, AA₁ et AA₄ ne sont pas absents simultanément ;
R1 et R2, différents ou identiques l'un à l'autre, sont choisis parmi : un Fab, un scFv, un nanocorps (NB), un anticorps et leurs combinaisons,
ou
lesdites nanoparticules d'albumine ayant la formule (II) : 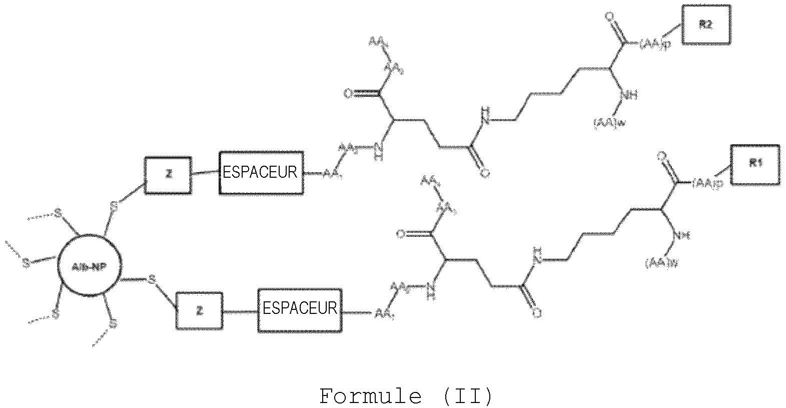 où,
Z-espaceur-AA₁-AA₂-Q-AA₃-AA₄ est un coupleur ;
Alb-NP, Z, l'espaceur, R1 et R2 sont tels que définis pour la formule (I) ;
AA₁-AA₂-Q-AA₃-AA₄, avec Q égal à la glutamine de formule -CO-(CH₂)₂-CH-(NH)-CO-, est tel que défini pour la formule (I) et est lié à l'espaceur au moyen d'une liaison amide entre le terminal -CO- de l'espaceur et un groupe -NH- de l'acide aminé AA₁, s'il est présent, ou AA₂ ;
la séquence peptidique contenant une lysine (K ; Lys) a la formule (IX) :
**(AA)_{w}-K-(A.A)ₚ** Formule (IX)
où
w et p sont des nombres entiers compris entre 0 et 8, de préférence entre 1 et 5, à condition que w et p ne soient jamais simultanément égaux à 0 ;
AA désigne un acide aminé choisi parmi : l'alanine (A ; Ala), la tyrosine (Y ; Tyr), la phénylalanine (F ; Phe), la glycine (G ; Gly), le tryptophane (W ; Trp) et la sérine (S ; Ser) ;
K est une lysine (Lys).

2. Nanoparticules d'albumine selon la revendication 1, les nanoparticules étant des nanoparticules d'albumine humaine (NP-HSA) ou des nanoparticules d'albumine bovine (NP-BSA).

3. Nanoparticules d'albumine selon la revendication 1 ou 2, les nanoparticules ayant un diamètre moyen (ou taille moyenne Z) de 100-500 nm, de préférence 100-400 nm ou 300-400 nm, mesuré par la technique de diffusion dynamique de la lumière (DLS).

4. Nanoparticules d'albumine selon l'une quelconque des revendications 1 à 3, chargées avec au moins un médicament cytotoxique choisi parmi : 5-FU, capécitabine, cytarabine, fludarabine, cladribine, paclitaxel, doxorubicine, daunorubicine, épirubicine, docétaxel, vinblastine, vincristine, vinorelbine, mercaptopurine, méthotrexate, raltitrexed, étoposide, téniposide, camptothécine, irinotécan, topotécan et leurs combinaisons.

5. Nanoparticules d'albumine selon l'une quelconque des revendications 1 à 4, R1 et R2 étant différents l'un de l'autre et étant deux types différents de Fab : Fabl et Fab2, deux types différents de scFv : scFv1 et scFv2, deux types différents de NB : NB1 et NB2, deux types différents d'anticorps : Ab1 et Ab2, ou R1 et R2 sont des combinaisons mixtes de molécules biologiques, de préférence Fab1 et scFv1, Fab1 et NB1, scFv1 et NB1, ou Ab1 et NB1.

6. Nanoparticules d'albumine selon l'une quelconque des revendications 1 à 5, l'anticorps étant un anticorps monoclonal choisi parmi : l'anticorps DI17E6, le Trastuzumab, le Pertuzumab, le Cetuximab, l'anticorps monoclonal anti-Cripto-1, de préférence l'anticorps 1B4 anti-Cripto-1 ou l'anticorps 10D1 antiCripto-1 ; le Fab est choisi parmi : le Fab recombinant de Trastuzumab, de Pertuzumab, d'un anticorps monoclonal anti-Cripto-1, de préférence choisi parmi l'anticorps 11B4 anti-Cripto-1 et l'anticorps 10D1 anti-Cripto-1 ; le scFv est un fragment fonctionnel d'un anticorps choisi parmi : l'anticorps DI17E6, le Trastuzumab, le Pertuzumab, le Cetuximab, un anticorps monoclonal antiCripto-1, de préférence l'anticorps 1B4 anti-Cripto-1 ou l'anticorps 10D1 anti-Cripto-1 ; les nanocorps (NB) sont choisis parmi un NB anti-VEGFR2, de préférence le NB 3VGR19 ; un NB anti-Her2, de préférence le NB 5F7GGC ; un NB anti-EGFR, de préférence le NB EGa1.

7. Nanoparticules d'albumine selon l'une quelconque des revendications 1 à 6, Z dérivant d'un groupe fonctionnel choisi parmi : l'acide 1-bromoacétique, l'acide 1-chloroacétique et l'acide 6-maléimidohexanoïque.

8. Nanoparticules d'albumine selon l'une quelconque des revendications 1 à 7, l'espaceur étant -NH-(CE₂-CH₂-O)ₙ-CH₂-CO-, n étant compris entre 2 et 5, ou est la glycine.

9. Nanoparticules d'albumine selon l'une quelconque des revendications 1 à 8, le groupe X-NH- étant choisi parmi : l'acide aminé L-lysine et l'acide aminé L-lysine amidé sur C-terminal.

10. Nanoparticules d'albumine selon l'une quelconque des revendications 1 à 9, la séquence consensus étant choisie parmi : LQSP, TQGA, LLQG.

11. Nanoparticules d'albumine selon l'une quelconque des revendications 1 à 8, où dans la formule (IX)
**(AA)_{w}-K-(A.A)ₚ**
w est égal à 0 et p est égal à 1-3 ; de préférence, w est égal à 0 et p est égal à 3, plus préférablement il s'agit de KAYA, KGYA, KSYA, KAFA, KGFA, KSFA, KAWA, KGWA, KSWA, KAYG, KGYG, KSYG, KAFG, KGFG, KSFG, KAWG, KGWG, KSWG, KAYS, KGYS, KSYS, KAFS, KGFS, KSFS, KAWS, KGWS, KSWS.

12. Nanoparticules d'albumine sérique selon l'une quelconque des revendications 1 à 11, les nanoparticules étant liées de manière covalente à la surface à un colorant fluorescent, de préférence l'isothiocyanate de fluorescéine.

13. Nanoparticules d'albumine sérique selon l'une quelconque des revendications 1 à 12, choisies parmi :

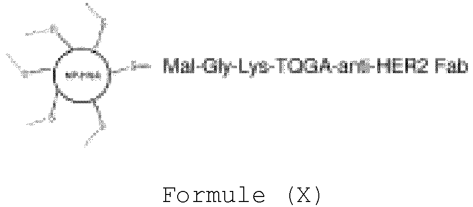

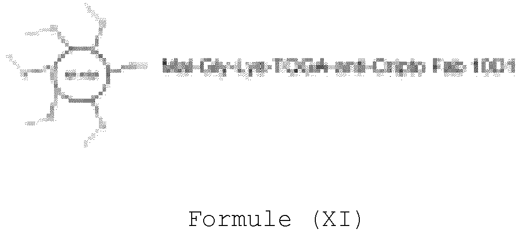

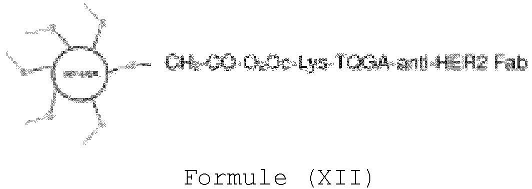

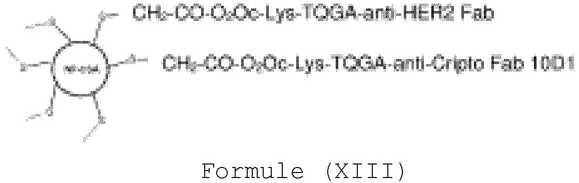

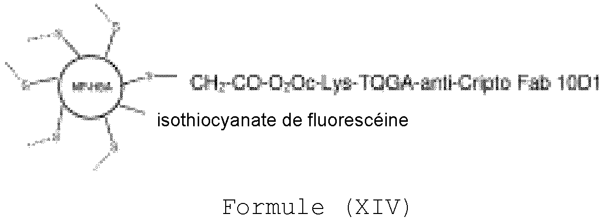

14. Procédé de préparation des nanoparticules d'albumine selon l'une quelconque des revendications 1 à 13, l'au moins une molécule biologique étant liée au coupleur par une liaison amide formée au moyen d'une réaction de transamidation (ou de transglutamination), médiée par l'enzyme transglutaminase, entre :
(i) le résidu NH₂ d'un groupe X d'un coupleur et le résidu -CO-NH₂ d'une glutamine comprise dans une séquence consensus peptidique insérée dans l'au moins une molécule biologique ;
ou
(ii) le résidu -NH₂ d'une lysine comprise dans une séquence peptidique insérée dans l'au moins une molécule biologique et le résidu -CO-NH₂ d'une glutamine insérée dans une séquence consensus faisant partie du coupleur.
